# FASCICULE DE BREVET EUROPEEN

(11) **EP 3 003 414 B1**
(45) Date de publication et mention de la délivrance du brevet: **16.10.2019**
(21) Numéro de dépôt: 14731810.9
(22) Date de dépôt: 03.06.2014
(51) Int. Cl.: A61L 27/58, A61L 27/54, A61L 27/24, A61L 27/22, A61L 27/10, A61L 27/56, A61L 27/30, A61L 27/18, A61L 27/16, A61L 27/20, A61L 27/44

(54) **IMPLANT A POROSITÉ CONTROLÉE EN UN MATÉRIAU HYBRIDE**
IMPLANTAT MIT KONTROLLIERTER POROSITÄT AUS EINEM HYBRIDMATERIAL
IMPLANT WITH CONTROLLED POROSITY MADE OF A HYBRID MATERIAL

(30) Priorité: 03.06.2013 FR 1355057
(43) Date de publication de la demande: 13.04.2016
(73) Titulaire: Université Clermont Auvergne, 63000 Clermont-Ferrand (FR); Centre National de la Recherche Scientifique (C.N.R.S.), 75794 Paris Cedex 16 (FR)
(72) Inventeur: LAO, Jonathan Claude Alexandre, 63960 Veyre-Monton (FR); LACROIX, Joséphine, 72000 Le Mans (FR); JALLOT, Edouard Daniel Albert, 63360 Saint-beauzire (FR); DIEUDONNE, Xavier, 63122 Ceyrat (FR)
(74) Mandataire: Gevers & Orès
(86) Numéro de dépôt international: PCT/IB2014/061914
(87) Numéro de publication internationale: WO 2014/195863

(56) Documents cités:
- JOAQUÍN RÓDENAS-ROCHINA ET AL: "Comparative study of PCL-HAp and PCL-bioglass composite scaffolds for bone tissue engineering", JOURNAL OF MATERIALS SCIENCE: MATERIALS IN MEDICINE, vol. 24, no. 5, 1 mai 2013 (2013-05-01), pages 1293-1308, XP055132431, ISSN: 0957-4530, DOI: 10.1007/s10856-013-4878-5
- FOROUGH HAFEZI ET AL: "Transplantation of nano-bioglass/gelatin scaffold in a non-autogenous setting for bone regeneration in a rabbit ulna", JOURNAL OF MATERIALS SCIENCE: MATERIALS IN MEDICINE, KLUWER ACADEMIC PUBLISHERS, BO, vol. 23, no. 11, 24 juillet 2012 (2012-07-24), pages 2783-2792, XP035135310, ISSN: 1573-4838, DOI: 10.1007/S10856-012-4722-3

## Description

L'invention concerne un matériau d'implant pour le comblement de défauts osseux, la régénération osseuse et l'ingénierie tissulaire de l'os, un implant comprenant ce matériau, un procédé de fabrication d'un tel implant ainsi qu'un procédé de fabrication d'un matériau hybride.

Le vieillissement global de la population et les troubles du système ostéo-articulaire qui l'accompagnent rendent nécessaire le développement de matériaux de substitution des tissus osseux de haute performance. 18 milliards d'euros de frais de santé sont en effet dépensés chaque année en France pour les maladies du système ostéo-articulaire et dentaires, les troubles musculo-squelettiques sont les pathologies professionnelles les plus répandues dans les pays industrialisés, tandis que l'ostéoporose se développe chez les patients âgés ; ces faits dessinent les contours d'un enjeu sociétal et économique majeur et expliquent la demande croissante en biomatériaux, implants à durée de vie accrue capables de combler les pertes osseuses.

Le recours aux greffes étant limité, et les matériaux d'origine animale pouvant poser des problèmes de biocompatibilité ou des risques d'infection, les efforts de recherche visent à élaborer des biomatériaux synthétiques capables de promouvoir la régénération osseuse.

On parle dans ce cas d'implants bioactifs : le matériau implanté n'est pas simplement destiné à combler de manière passive une perte osseuse en restant le plus inerte possible, mais au contraire il doit stimuler et participer activement au mécanisme de régénération osseuse. Ceci est particulièrement important dans le cas de larges défauts osseux, pour lesquels le mécanisme d'autoréparation ne fonctionne plus.

Actuellement les principaux matériaux bioactifs utilisés comme substituts osseux sont les « céramiques » bioactives, telles que les phosphates de calcium, et les verres bioactifs, également appelés « bioverres ».

Les premières céramiques bioactives ont été développées par L.L. Hench (L.L. Hench et al., J. Biomed. Mater. Res. 1971, 2, 117-141 ; L.L. Hench et al., J. Biomed. Mater. Res. 1973, 7, 25-42).

Les premiers verres bioactifs ont été préparés à partir de SiO₂, de P₂O₅, de CaO et de Na₂O. Les oxydes de silicium et de phosphore sont des formateurs de réseau qui participent à la cohésion du réseau vitreux. Les alcalins et alcalino terreux comme le sodium et le calcium ne présentent pas cette capacité et viennent modifier le réseau vitreux en y introduisant des ruptures de chaines qui sont à l'origine de la faible température de fusion de ces verres associée à un désordre structural accru. Leur présence a pour conséquence une plus grande réactivité des verres bioactifs à travers notamment leur corrosion dans un environnement aqueux. Cette réactivité permet la formation d'hydroxyapatite en milieu physiologique et favorise donc la reconstruction osseuse.

Le bioverre qui a été le plus étudié est un verre sodo-silico-phosphocalcique dit Bioglass® ou Bioverre de Hench. Sa composition de base est 45% SiO₂ - 24,5% CaO - 24,5% Na₂O - 6% P₂O₅, en masse par rapport à la masse totale de la composition. Les propriétés bioactives remarquables de ce matériau ne sont plus à démontrer. Le Bioglass® reste à l'heure actuelle un des matériaux bioactifs (induisant une réponse spécifique des cellules) les plus intéressants.

De nombreux développements ont été faits dans le domaine des verres bioactifs depuis leur découverte (M. Vallet-Regi et al., Eur. J. Inorg. Chem. 2003, 1029-1042), tels que l'incorporation de différents atomes ou l'incorporation de principes actifs. Les compositions des verres bioactifs ont été optimisées de façon à favoriser la prolifération des ostéoblastes et la formation de tissus osseux (WO 02/04606). L'incorporation d'argent a été proposée notamment pour conférer des propriétés antibactériennes aux verres bioactifs (WO 00/76486).

La demande WO 2009/027594 décrit, elle, un verre bioactif dans lequel le strontium est introduit en des quantités comprises entre 0,1 à 10% du poids total du verre bioactif. J. Rodenas-Rochina et al ,J. of Materials science in medecine, 2013, 1293-1308, décrit une étude montrant la possibilité de former des implants osseux. Des particules de polyméthacrylate d'éthyl (PEMA) ayant un diamètre de 200 µm sont utilisées comme agent porogène, elle sont mélangées à une solution de polycaprolactone dans du dioxane contenant des particules de verre de 20 µm de diamètre. Le PEMA est dissous par ajout d'éthanol. La solution de polymère contient entre 5 et 20% de particule de verre et le rapport PEMA/polycaprolactone est de 1:1 en poids.

Ces matériaux bioactifs présentent comme caractéristique d'être tout à la fois biocompatibles, capables de se lier spontanément aux tissus osseux, de promouvoir l'adhésion des cellules osseuses et, enfin d'être biorésorbables, étant progressivement remplacés par du tissu osseux néoformé à mesure que la repousse osseuse avance.

Cependant, malgré cet ensemble de caractéristiques très satisfaisantes, la fragilité de ces matériaux en limite les applications : en effet, bien que leur rigidité soit souvent supérieure à celles de l'os, leur manque de souplesse et de ténacité font que les matériaux bioactifs ne peuvent être implantés en des sites mécaniquement chargés.

Pour pallier ce défaut, une solution ingénieuse est de s'inspirer de la structure particulière du tissu osseux. Complexe, celle-ci consiste principalement en une trame composite mêlant intimement une phase inorganique, le minéral osseux constitué de cristaux d'apatite (phosphate de calcium résorbable), à une phase organique, qui est majoritairement du collagène. De manière remarquable, une telle structure composite associe la rigidité initiale de la partie inorganique à la ténacité et à la souplesse naturelle des fibres de collagène. Pour obtenir des implants aux propriétés mécaniques proches du tissu osseux, une stratégie consiste donc à combiner matériaux bioactifs et polymères biodégradables au sein d'une même matrice composite ou hybride.

Pour le comblement de larges défauts osseux, les implants doivent avoir, en plus des caractéristiques précédentes, une morphologie spécifique : celle-ci s'inspire de l'os trabéculaire, à savoir une structure hautement poreuse constituée d'un réseau tridimensionnel de macropores interconnectés de plusieurs centaines de microns. En effet, dans le cas de larges défauts osseux, les cellules de l'os ont besoin d'une matrice "support" extracellulaire capable de guider et de stimuler l'adhésion, la prolifération, la différentiation cellulaire, tout en étant compatible avec les processus de vascularisation et d'invasion tissulaire.

Une telle structure macroporeuse est également requise pour les nouvelles applications envisagées en ingénierie tissulaire de l'os: il s'agit, à partir de cellules prélevées chez le patient, de fabriquer en laboratoire du tissu osseux nouveau que l'on pourra ré-implanter a posteriori chez le patient. Pour être conduite de façon optimale, cette culture de tissu doit là aussi reposer sur des supports tridimensionnels poreux permettant une bonne adhésion cellulaire, la différentiation en cellules matures ainsi que la fabrication du tissu et en particulier la biominéralisation.

En résumé, si de nombreux matériaux et formulations ont été développés pour le comblement des pertes osseuses, aucun ne répond totalement au cahier des charges décrivant l'implant idéal, à savoir :
- être biocompatible ;
- être bioactif : induire spontanément la formation d'un lien interfacial fort avec les tissus osseux, promouvoir l'adhésion et l'activité cellulaire ;
- être biorésorbable ;
- avoir une morphologie adéquate basée sur une matrice tridimensionnelle de macropores interconnectés ;
- avoir une bonne tenue mécanique ;
- être dérivé d'un procédé de fabrication permettant une mise en forme facile et suffisamment souple pour s'adapter à de nombreuses géométries de défauts.

Par morphologie adéquate basée sur une matrice tridimensionnelle de macropores interconnectés, on entend que la taille, la forme et la distribution des pores ainsi que la taille des interconnexions entre ces pores doivent être contrôlées.

L'invention a pour but de proposer un matériau qui répond parfaitement à tous ces critères et qui peut être fabriqué par un procédé qui permet la réalisation d'architectures poreuses composées d'une partie inorganique et d'une partie organique, sous forme de matériau hybride, au contraire des procédés de l'art antérieur.

A cet effet, l'invention propose un procédé de fabrication d'un implant en un matériau hybride pour le comblement de défauts osseux, la régénération osseuse et l'ingénierie tissulaire de l'os, caractérisé en ce qu'il comprend les étapes suivantes :
a) sélection d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S différent du solvant S1,
c) sélection de microsphères d'un agent porogène A ayant des diamètres et des tailles correspondant aux diamètres et tailles recherchés des pores dans le matériau constituant l'implant à fabriquer, cet agent porogène A étant :
   - en un polymère insoluble dans le au moins un solvant S1 et soluble dans le au moins un solvant S,
      le au moins un solvant S dans lequel le matériau du polymère biodégradable P est insoluble et le au moins un solvant S dans lequel le matériau de l'agent porogène A est soluble étant identiques,
d) introduction d'au moins 60% en volume, de préférence 70% en volume par rapport au volume total du mélange agent porogène A-polymère biodégradable P-précurseurs alcoxydes du verre bioactif M, de microsphères de l'agent porogène A dans un moule ayant la forme et la taille recherchées pour l'implant, ces microsphères formant un empilement compact correspondant à la forme et à la taille des pores à obtenir dans le matériau d'implant,
e) introduction du polymère biodégradable P dans les précurseurs alcoxydes du verre bioactif M et homogénéisation du mélange,
f) introduction du mélange obtenu à l'étape e) dans le moule,
g) gélification du mélange contenu dans le moule après l'étape f),
h) démoulage du mélange obtenu à l'étape g),
i) élimination des microsphères d'agent porogène A par lavage avec le solvant S.

Dans un premier mode de mise en oeuvre du procédé de l'invention, l'étape e) et/ou l'étape f) sont mises en oeuvre avant l'étape d).

Dans un second mode de mise en oeuvre du procédé de l'invention, les étapes d) et e) et f) sont mises en oeuvres simultanément.

Dans tous les modes de mise en oeuvre, le procédé de l'invention peut comprendre, de plus, une étape j) de réticulation du matériau obtenu à l'étape i).

Egalement dans tous les modes de mise en oeuvre du procédé de l'invention, le polymère biodégradable P est choisi parmi :
- les polymères biodégradables solubles dans au moins un solvant S1 et insolubles dans au moins un solvant S choisis parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables de préférence l'alcool polyvinylique (PVA) ou le poly(acide lactique) (PLA),
- les polymères synthétiques biodégradables, de préférence un polyéthylène glycol (PEG), ou le poly(caprolactone) (PCL),
- les protéines, de préférence la gélatine ou le collagène,
et le matériau de l'agent porogène A est choisi parmi les polymères biodégradables insolubles dans le au moins un solvant S1 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène, l'acrylonitrile butadiène styrène (ABS),
le matériau de l'agent porogène A étant différent du polymère biodégradable P.

De préférence, le rapport en poids polymère biodégradable P/verre bioactif M est compris 10/90 et 90/10, de préférence, pour des raisons de tenue mécanique du matériau obtenu, il sera compris entre 20/80 et 80/20, la meilleure tenue mécanique (manipulation aisée sans déformations ou perte de matière) étant obtenue avec un rapport 70/30.

Egalement de préférence, le verre bioactif M est un verre à base de SiO₂ et de CaO, le polymère biodégradable P est la gélatine, le matériau des microsphères d'agent porogène A est le polyméthacrylate de méthyle et le solvant S est l'acétone.

Le procédé de l'invention peut comprendre, de plus, à l'étape f), une étape d'introduction d'un agent de couplage, de préférence un composé organoalkoxysilane, plus préférablement du 3-glycidoxypropyltriméthoxysilane (GPTMS), encore plus préférablement du 3-glycidoxypropyltriéthoxysilane (GPTES).

Il peut également comprendre, de plus, après l'étape d), une étape d'agrandissement des interconnexions, par infiltration d'un solvant S du matériau de l'agent porogène A, dans l'empilement des microsphères d'agent porogène A et/ou par chauffage de cet empilement.

L'invention propose également un procédé de fabrication d'un matériau hybride comprenant un polymère biodégradable P et un verre bioactif M à base de SiO₂ et de CaO et contenant optionnellement du P₂O₅ et/ou optionnellement dopé au strontium caractérisé en ce qu'il comprend les étapes suivantes :
A) préparation d'un sol des précurseurs alcoxydes d'un verre bioactif M à base de SiO₂ et de CaO, contenant optionnellement P₂O₅ et/ou optionnellement dopé au strontium,
B) ajout d'une solution contenant le polymère biodégradable P, dissout dans un solvant S1, dans le sol de l'étape A),
C) polymérisation sol-gel des précurseurs alcoxydes et gélification du mélange obtenu à l'étape B) à une température comprise entre 0°C et 60°C, sous air.

L'invention propose encore un matériau d'implant pour le comblement de défauts osseux, la régénération osseuse et l'ingénierie tissulaire de l'os,
caractérisé en ce qu'il comprend un matériau hybride comprenant :
- un verre bioactif M à base de SiO₂ et CaO, optionnellement contenant P₂O₅ et/ou optionnellement dopé au strontium, et
- un polymère biodégradable P soluble dans le au moins un solvant S1 choisi parmi :
   - les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
   - les polyesters biorésorbables, de préférence l'alcool polyvinylique ou le poly(acide lactique),
   - les polymères synthétiques biodégradables, de préférence un polyéthylène glycol, ou le poly(caprolactone), et
   - les protéines, de préférence la gélatine ou le collagène,
et en ce que il consiste en une matrice comprenant le matériau hybride, cette matrice ayant au moins 70% en nombre de pores ayant la forme de sphères ou de polyèdres sphériques s'inscrivant dans une sphère, le diamètre des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions entre les pores ayant leur plus petite dimension comprise entre 25 µm et 250 µm, bornes incluses, au moins 70% en nombre de ces pores ayant au moins une interconnexion avec un autre pore.

L'invention propose enfin un implant pour le comblement de défauts osseux, la régénération osseuse et l'ingénierie tissulaire de l'os, caractérisé en ce qu'il comprend un matériau selon l'invention ou obtenu par le procédé de fabrication d'un implant selon l'invention.

L'invention sera mieux comprise et d'autres caractéristiques et avantages de celle-ci apparaîtront plus clairement à la lecture de la description explicative qui suit et qui est faite en référence aux figures annexées dans lesquelles :
- la figure 1 représente une vue en coupe prise au microscope électronique à balayage de l'implant en matériau composite obtenu à l'exemple 1, à un grandissement de ×70,
- la figure 2 est une représentation schématique du matériau d'implant selon l'invention,
- la figure 3 représente une photographie prise au microscope électronique à balayage de l'implant obtenu à l'exemple 3, dont la matrice est constituée de gélatine recouverte d'un verre bioactif, à un grandissement de ×100,
- la figure 4 représente une photographie prise au microscope électronique à balayage, à un grandissement de ×50 d'une coupe d'un matériau d'implant de l'art antérieur préparé par un procédé de lyophilisation, décrit dans Kim et al. "Hydroxyapatite and gelatin composite foams processed via novel freeze-drying and crosslinking for use as temporary hard tissue scaffolds" J Biomed Mater Res 72A: 136-145, 2005
- la figure 5 représente une photographie prise au microscope électronique à balayage à un grandissement de x200, d'un matériau d'implant de l'art antérieur préparé par un procédé de séparation de phases induite thermiquement, décrit dans Blaker et al. "Mechanical properties of highly porous PDLLA/Bioglass® composite foams as scaffolds for bone tissue engineering" Acta Biomater 2005, 1, 643-52,
- la figure 6 représente une photographie prise au microscope électronique à balayage à un grandissement de ×50, d'un matériau d'implant hybride selon l'invention de rapport massique gélatine/verre 70/30, obtenu par le procédé de l'invention comprenant une étape d'augmentation de la taille des interconnexions entre pores par infiltration avec un mélange acétone-éthanol à 30% en volume d'acétone, par rapport au volume total du mélange pendant 5 minutes par infiltration avec le mélange dans l'empilement des microsphères d'agent porogène, seul,
- la figure 7 est une photographie prise au microscope électronique à balayage à un grandissement de x50 du même matériau d'implant selon l'invention que représenté en figure 6 mais après infiltration avec un mélange acétone-éthanol à 30% en volume d'acétone, par rapport au volume total du mélange, pendant 15 minutes, avec le mélange dans l'empilement des microsphères d'agent porogène, seul,
- la figure 8 est une photographie prise au microscope électronique à balayage à un grandissement de × 50 du matériau représenté en figure 6 et en figure 7 obtenu par le procédé de l'invention après augmentation de la taille des interconnexions entre pores par infiltration du mélange acétone-éthanol à 30% en volume d'acétone, par rapport au volume total du mélange, pendant 30 minutes, avec le mélange dans l'empilement des microsphères d'agent porogène, seul,
- la figure 9 est une courbe représentant l'augmentation de la taille des interconnexions entre pores par infiltration avec un mélange acétone-éthanol à 30% en volume d'acétone, par rapport au volume total du mélange, en fonction de ce temps d'infiltration,
- la figure 10 est une photographie prise au microscope électronique à balayage à un grandissement de ×100 d'un matériau d'implant hybride selon l'invention constitué de 70% de gélatine et 30 % de verre, en masse, selon l'invention obtenu par le procédé de l'invention après augmentation de la taille des interconnexions des pores par chauffage de l'empilement des microsphères d'agent porogène, seul, à 125°C pendant 15 minutes, sous air,
- la figure 11 est une photographie prise au microscope électronique à balayage à un grandissement de ×100 du matériau d'implant représenté en figure 10 mais après augmentation de la taille des interconnexions de pores par chauffage à 125°C pendant 1 heure de l'empilement de microsphères d'agent porogène, seul, avant infiltration avec le matériau hybride constitué de 70% de gélatine et 30 % de verre, en masse,
- la figure 12 est une photographie prise au microscope électronique à balayage à un grandissement de ×100 de la même composition de matériau d'implant selon l'invention que représenté en figure 10 et 11, obtenu par le procédé de l'invention après augmentation de la taille des interconnexions de pores par chauffage à 125°C de l'empilement de microsphères d'agent porogène, seul, pendant 2 heures,
- la figure 13 représente la courbe montrant l'évolution de la taille des interconnexions entre pores en fonction du temps de chauffage à 125°C de l'empilement de microsphères d'agent porogène, seul,
- la figure 14A est une photographie prise au microscope électronique à balayage à un grandissement de ×100 du matériau hybride obtenu à l'exemple 16,
- la figure 14B montre le spectre PIXE (Particles or Protons Induced X-ray Emission, en anglais) du matériau hybride obtenu à l'exemple 16,
- la figure 15 est une photographie prise au microscope électronique à balayage à un grandissement de ×100 du matériau hybride selon l'invention obtenu à l'exemple 7,
- la figure 16 est une photographie prise au microscope électronique à balayage à un grandissement de ×100 du matériau d'implant selon l'invention obtenu à l'exemple 8,
- la figure 17 est une photographie prise au microscope électronique à balayage à un grandissement de x30 du matériau d'implant selon l'invention obtenu à l'exemple 9,
- la figure 18 est une photographie prise au microscope électronique à balayage à un grandissement de ×100 du matériau d'implant selon l'invention obtenu à l'exemple 10,
- la figure 19 est une photographie prise au microscope électronique à balayage à un grandissement de ×70 du matériau d'implant selon l'invention obtenu à l'exemple 11,
- la figure 20 est une photographie prise au microscope électronique à balayage à un grandissement de ×30 du matériau d'implant selon l'invention obtenu à l'exemple 12,
- la figure 21 est une photographie prise au microscope électronique à balayage à un grandissement de ×70 du matériau d'implant selon l'invention obtenu à l'exemple 13, et
- la figure 22 est une photographie prise au microscope électronique à balayage à un grandissement de ×30 du matériau d'implant selon l'invention obtenu à l'exemple 14.

Dans ce qui précède et ce qui suit, les termes suivants ont les définitions suivantes :
- "interconnexion(s) entre pores" : ouverture(s) permettant le passage d'un pore à l'autre,
- "milieu aqueux" : tout milieu liquide contenant de l'eau, ou eau seule,
- "biodégradable" : dégradable dans un liquide physiologique, par exemple une solution saline tamponnée (SBF),
- "biorésorbable" : éliminable dans un milieu physiologique contenant des cellules biologiques,
- "diamètre moyen arithmétique de l'ensemble des pores" : somme des diamètres des pores / nombre de pores,
- "pore sphérique" ou "sphère" : pore ou sphère dont le rapport du plus petit diamètre sur le plus grand diamètre est de 0,9 ± 0,1,
- "polyèdre s'inscrivant dans une sphère" : polyèdre s'inscrivant dans une sphère ayant en tous points le même diamètre, les différences entre les différents diamètres du polyèdre s'inscrivant dans cette sphère étant d'au plus ± 15% du diamètre de la sphère dans laquelle ils s'inscrivent,
- "empilement compact de microsphères d'agent porogène A" : empilement de microsphères d'agent porogène A dans lequel :
   au moins 70% en nombre, de préférence plus de 95% en nombre de microsphères sont en contact les unes avec les autres, et restent en contact les unes avec les autres lorsque le mélange agent porogène A et hybride polymère biodégradable P-verre bioactif M-sont dans le moule, et lorsque l'empilement de microsphères est recouvert et infiltré avec le mélange hybride verre bioactif M-polymère biodégradable P.

On peut obtenir un tel empilement compact de microsphères d'agent porogène A par centrifugation du mélange microsphères d'agent porogène A et hybride polymère biodégradable P-verre bioactif M ou encore en appliquant une pression négative (vide) ou positive (supérieure à la pression atmosphérique) sur le mélange microsphères d'agent porogène A et hybride polymère biodégradable P-verre bioactif M introduit dans le moule, avant et pendant la gélification de ce mélange.

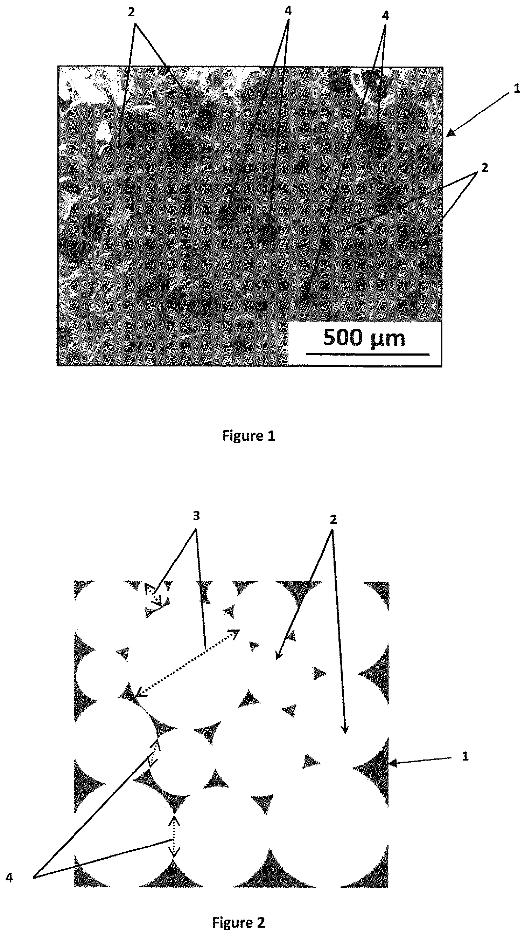

Le matériau d'implant pour le comblement de défauts osseux, la régénération osseuse et l'ingénierie tissulaire de l'os sera décrit en relation avec les figures 1 et 2.

Comme on le voit en figures 1 et 2, le matériau d'implant de l'invention comprend une matrice, notée 1 en figures 1 et 2, en un matériau qui comprend une partie organique et une partie inorganique.

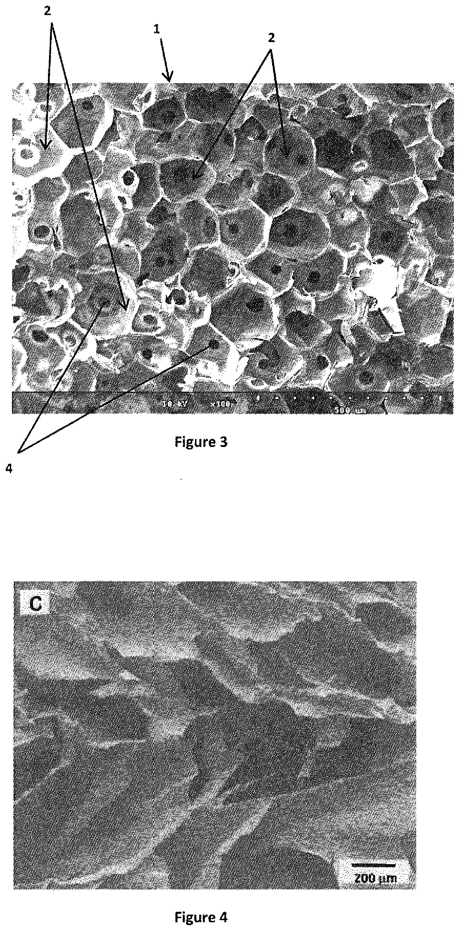

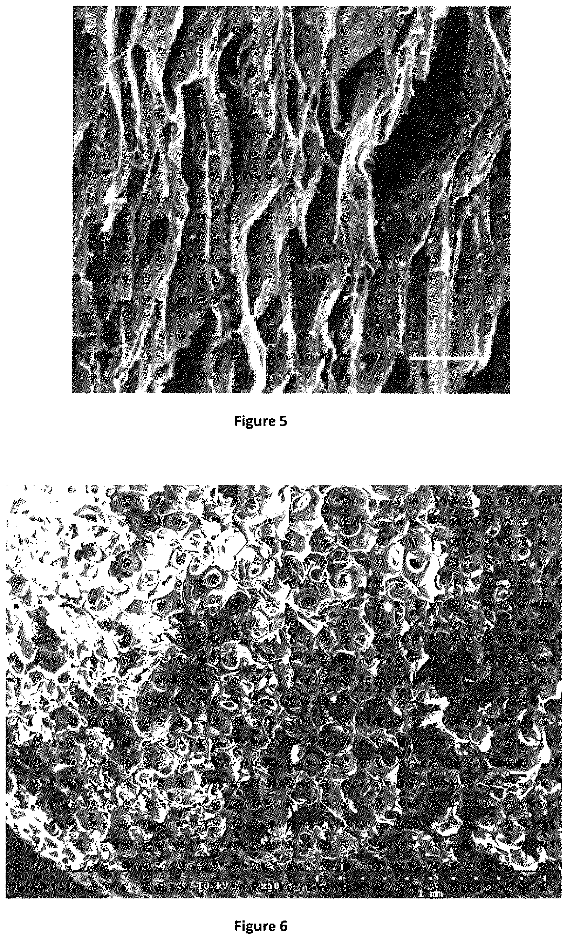

Ce matériau est biocompatible, bioactif, biorésorbable et comme on le voit sur les figures 1 à 3, il a une morphologie très régulière, en termes de distribution de pores, en termes de forme de pores, au contraire des matériaux de l'art antérieur qui ont une distribution, une taille et une forme de pores anarchiques, comme on peut le voir en figures 4 et 5 qui représentent respectivement des photographies prises au microscope électronique à balayage de matériaux d'implant obtenus par un procédé de lyophilisation (figure 4) et un procédé de séparation de phases induite thermiquement (figure 5).

En particulier, ce matériau a des pores, notés 2 en figures 1 à 3, en forme de sphères dont le diamètre, noté 3 en figure 2, soit est identique en tous points, soit en forme de sphères dont le rapport du plus petit diamètre au plus grand diamètre est de 0,9 ± 0,1, au maximum, soit en forme de polyèdres s'inscrivant dans une telle sphère, les différences entre les diamètres en différents points du polyèdre s'inscrivant dans cette sphère étant d'au plus plus ou moins 15 % du diamètre de la sphère dans laquelle ils s'inscrivent.

Les matériaux d'implant de l'invention peuvent avoir des tailles de pores se situant dans un très large intervalle de 100 à 900 µm, de préférence 200 µm à 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant en association avec des interconnexions, notées 4 en figures 1 à 3, entre pores dont la plus petite dimension est comprise entre 25 µm et 250 µm, bornes incluses.

Au moins 70% en nombre de ces pores a au moins une interconnexion avec un autre pore.

Ainsi, une telle forme et distribution de tailles de pores ainsi que de telles tailles d'interconnexions entre pores sont très favorables à la conduction des cellules, à la repousse osseuse et à l'invasion tissulaire comme cela a été démontré par Karageorgiou et al., "Porosity of 3D biomaterial scaffolds and osteogenesis". Biomaterials 2005, 26, (27), 5474-5491.

Cependant, dans le cas de cet article, de telles formes de pores avaient été obtenues sur un implant entièrement en céramique bioactive, c'est-à-dire en un phosphate de calcium (hydroxyapatite).

Or, de telles céramiques ont l'inconvénient de ne pas présenter la flexibilité requise pour un implant osseux. Leur procédé de fabrication ne peut pas être appliqué à un matériau comprenant une partie organique, comme celui de l'invention, car il implique une étape de frittage à des températures de l'ordre de 800°C, auxquelles la partie organique se désintègre.

De telles distributions de tailles ne sont jamais atteintes dans les matériaux d'implants comprenant une partie organique et une partie inorganique issus des procédés de l'art antérieur pour lesquels les pores ont des tailles généralement bien inférieures à 200 µm avec des interconnexions de tailles bien inférieures.

Il existe bien des matériaux d'implants issus des procédés de moussage mais ceux-ci ont alors des distributions de tailles de pores et d'interconnexions très larges, non contrôlées, la porosité pouvant même atteindre le millimètre, ce qui est n'est pas favorable à la tenue mécanique de l'implant.

WO 2013/023064 décrit un procédé pour obtenir une matrice dont la taille permet l'infiltration des cellules et la croissance interne d'os. Cette matrice peut être une matrice fibreuse qui n'a donc pas de pores sphériques tels que définis dans la présente invention, ou encore une matrice obtenue par moulage par solvant. Cependant, l'exemple 1B de ce document décrivant l'obtention d'une matrice par moulage par solvant n'a pas pu être reproduit en raison de la quantité trop importante d'agent porogène (90% V/V de particules de NaCl) utilisée qui ne permet pas l'obtention d'un mélange homogène avec le verre bioactif et le polymère biodégradable.

Comme on le verra par la suite, grâce au procédé de fabrication d'un implant hybride selon l'invention, il est possible de contrôler la dispersion du jeu de tailles de pores et d'interconnexions de la matrice, ce qui n'était pas possible dans les procédés de l'art antérieur où la porosité générée est distribuée de manière aléatoire dans leurs intervalles respectifs.

La matrice 1 est constituée d'une phase organique et d'une phase inorganique.

La phase inorganique est un verre bioactif M.

Les céramiques bioactives et les verres bioactifs sont bien connus de l'homme du métier et sont décrits en particulier dans L.L. Hench et al., J. Biomed. Mater. Res. 1971, 2, 117-141 ; L.L. Hench et al., J. Biomed. Mater. Res. 1973, 7, 25-42 pour les céramiques bioactives et dans M. Vallet-Regi et al., Eur. J. Inorg. Chem. 2003, 1029-1042 et WO 02/04606, WO 00/76486 et WO 2009/027594, en particulier. Dans l'invention, on utilise uniquement un verre bioactif.

La partie organique du matériau d'implant de l'invention est un polymère biodégradable P soluble dans au moins un solvant S1 et insoluble dans au moins un solvant S. Ces solvants peuvent être de l'eau, un milieu aqueux ou encore un solvant organique. De préférence, le polymère biodégradable P est choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone), et
- les protéines, de préférence la gélatine ou le collagène.

Dans les matériaux d'implant, la matrice 1 peut consister en le verre bioactif M et le polymère biodégradable P qui forment un matériau composite, c'est-à-dire que les deux phases verre bioactif M et polymère biodégradable P coexistent dans l'architecture de la matrice. Ce matériau ne fait pas partie de la présente invention.

En effet, la matrice 1 du matériau d'implant de l'invention est constituée du verre bioactif M et du polymère biodégradable P qui forment un matériau hybride, c'est-à-dire formant une seule phase.

Le matériau hybride utilisé dans l'invention est obtenu par un procédé, qui est aussi un objet de l'invention, qui comprend la formation d'un sol contenant tous les précurseurs alcoxydes du verre bioactif, l'ajout du polymère biodégradable P dans ce sol et la gélification de la solution ainsi obtenue par une succession de réactions de polymérisation (polymérisation sol-gel de la phase inorganique) (condensation des alcoxydes). On obtient alors un mélange hybride associant intimement la phase minérale et la phase organique.

Le matériau hybride se distingue donc du matériau composite par une intégration intime entre les deux phases organique et inorganique, ces deux phases étant indiscernables (sauf à l'échelle moléculaire) dans le cas d'un mélange hybride.

Mais, la matrice 1 peut également être formée du seul polymère biodégradable P, polymère qui est recouvert du verre bioactif M, par exemple par imprégnation de la matrice 1 en polymère biodégradable P dans une suspension du verre bioactif M ou encore en immergeant la matrice 1 uniquement formée du polymère biodégradable P dans un sol contenant tous les précurseurs du verre bioactif M.

Dans les deux cas, la matrice 1 sera ensuite séchée pour permettre le dépôt des particules de verre bioactif M ou la gélification du sol selon le cas. Ce mode de réalisation n'est également pas un objet de l'invention.

Le matériau d'implant de l'invention est obtenu par un procédé faisant appel à un agent porogène A qui est constitué de microsphères en un polymère soluble dans au moins un solvant S dans lequel le polymère biodégradable P n'est, lui, pas soluble.

Ainsi, le procédé de l'invention consiste à empiler des microsphères d'agent porogène A en un matériau polymère, différent du polymère biodégradable P, dans un moule ayant la forme et la taille correspondant à la géométrie du défaut osseux à combler ou du défaut où la régénération osseuse est voulue.

Ces microsphères d'agent porogène A permettent d'obtenir au final des pores dont la taille et la distribution correspondront en négatif à l'empilement de microsphères d'agent porogène A initialement réalisé.

De plus, au moins 70 % en nombre de pores formés auront la forme de sphères parfaites, c'est-à-dire auront soit en tous points un diamètre égal, soit la forme de sphères dont le rapport du plus petit diamètre au plus grand diamètre est de 0,9 ± 0,1, au maximum, ou pour les plus grands pores, auront la forme d'un polyèdre s'inscrivant dans une sphère ayant en tous points le même diamètre, les différences entre les diamètres en différents points du polyèdre s'inscrivant dans cette sphère étant d'au plus plus ou moins 15 % du diamètre de la sphère dans laquelle ils s'inscrivent.

En effet, le matériau destiné à constituer la matrice 1 sera ensuite infiltré dans l'empilement des billes de microsphères d'agents porogènes A, puis solidifié pour pouvoir être démoulé sans changer la forme et la taille de l'empilement de l'implant voulu. L'agent porogène A sera alors éliminé permettant l'obtention du matériau d'implant de l'invention.

Comme on le voit, ce procédé n'utilise aucun traitement thermique à haute température pour fritter le verre bioactif M, la seule température nécessaire étant la température d'évaporation du solvant S utilisé.

Il en est de même pour le cas où la matrice 1 est constituée uniquement du polymère biodégradable P qui est ensuite recouvert du verre bioactif M.

Comme cela apparaîtra clairement, l'invention réside sur la judicieuse association du choix de trois matériaux : le matériau constituant le polymère biodégradable P, le matériau constituant l'agent porogène A et le solvant S de l'agent porogène A qui ne doit pas dissoudre le polymère biodégradable P.

Le matériau du polymère biodégradable P qui fait partie du matériau d'implant doit être un polymère biocompatible.

Le matériau de l'agent porogène A, doit être, lui, un matériau, par exemple un polymère, dont le solvant S est un non-solvant du polymère biodégradable P.

On comprend alors que le choix de l'un des trois éléments du trio "polymère biodégradable P-agent porogène A-solvant S du porogène" ne peut se faire indépendamment du choix des autres.

Les polymères biodégradables P doivent être solubles dans au moins un solvant S1, qui peut être de l'eau ou des solutions aqueuses ou encore un solvant organique, et insolubles dans au moins un solvant S différent du solvant S1. Ce solvant S peut lui aussi être de l'eau, un milieu aqueux ou un solvant organique.

Parmi les polymères biodégradables P préférés utilisables se trouvent :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine ;
- les polyesters biorésorbables, de préférence l'alcool polyvinylique; ou l'acide polylactique,
- les polymères synthétiques biodégradables, de préférence un polyéthylène glycol, ou le polycaprolactone, et
- les protéines, de préférence la gélatine ou le collagène

Le matériau constituant l'agent porogène A doit être soluble dans le au moins un solvant S dans lequel le polymère biodégradable P est insoluble.

Des exemples de tels matériaux sont les polymères biodégradables insolubles dans un milieu aqueux et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène, ou l'acrylonitrile butadiène styrène (ABS).

Dans tous les cas, le solvant S du matériau de l'agent porogène A ne devra pas être un solvant pour le matériau choisi pour servir de polymère biodégradable P et, bien entendu, le matériau de l'agent porogène A devra être différent du polymère biodégradable P.

Les solvants S sont en particulier l'acétone, l'éthanol, le chloroforme, le dichlorométhane, l'hexane, le cyclohexane, le benzène, l'éther diéthylique, l'hexafluoroisopropanol.

Dans l'invention, de préférence, le polymère biodégradable P sera la gélatine, les microsphères d'agent porogène A seront en polyméthacrylate de méthyle et le solvant S sera l'acétone.

Dans le procédé de fabrication du matériau d'implant hybride de l'invention, l'introduction des microsphères dans le moule peut être faite avant l'introduction du mélange des précurseurs alcoxydes du verre bioactif M et du polymère biodégradable P.

Cependant, il est également possible de d'abord introduire le mélange des précurseurs alcoxydes du verre bioactif M et du polymère biodégradable P dans le moule, et d'y verser ensuite les microsphères d'agent porogène A.

Il est encore possible de réaliser un mélange des précurseurs alcoxydes du verre bioactif M, du polymère biodégradable P et des microsphères d'agent porogène A et d'introduire le tout dans le moule.

Pour obtenir un matériau dont au moins 70% en nombre de pores, ont au moins une interconnexion avec un autre pore, la quantité d'agent porogène A introduite dans le mélange polymère biodégradable P-verre bioactif M doit représenter au moins 60% en volume du volume total du mélange polymère biodégradable P-verre bioactif M-agent porogène A introduit dans le moule.

La taille des interconnexions est liée à la taille du point de contact entre sphères d'agent porogène A dans l'empilement de sphères réalisé. L'augmentation de la taille des interconnexions générées, à diamètre poreux constant, est possible moyennant l'ajout d'une étape consistant en une fusion partielle des sphères porogènes dans l'empilement initialement réalisé, de manière à augmenter la taille de leur point de contact.

Les microsphères d'agent porogène A doivent former un empilement compact, lorsque placées dans le moule avec, dans l'invention, le sol des précurseurs alcoxydes du verre bioactif M et le polymère biodégradable P.

Pour cela, le volume d'agent porogène A, par rapport au volume total du mélange polymère biodégradable P-précurseurs du verre bioactif M-agent porogène A, doit être d'au moins 60%, de préférence d'au moins 70%.

Quant au rapport en masse biopolymère P/verre bioactif M, il pourra être compris entre 10/90 et 90/10. De préférence, pour des raisons de tenue mécanique du matériau obtenu, il sera compris entre 20/80 et 80/20, la meilleure tenue mécanique (manipulation aisée sans déformations ou perte de matière) étant obtenue avec un rapport 70/30.

Ce fusionnement peut se faire par infiltration du solvant S de l'agent porogène A sur l'empilement de l'agent porogène A, ou bien par échauffement de l'empilement des microsphères d'agent porogène A réalisé, ou bien des deux à la fois, de manière à réaliser la dissolution superficielle des sphères et permettre leur fusionnement partiel.

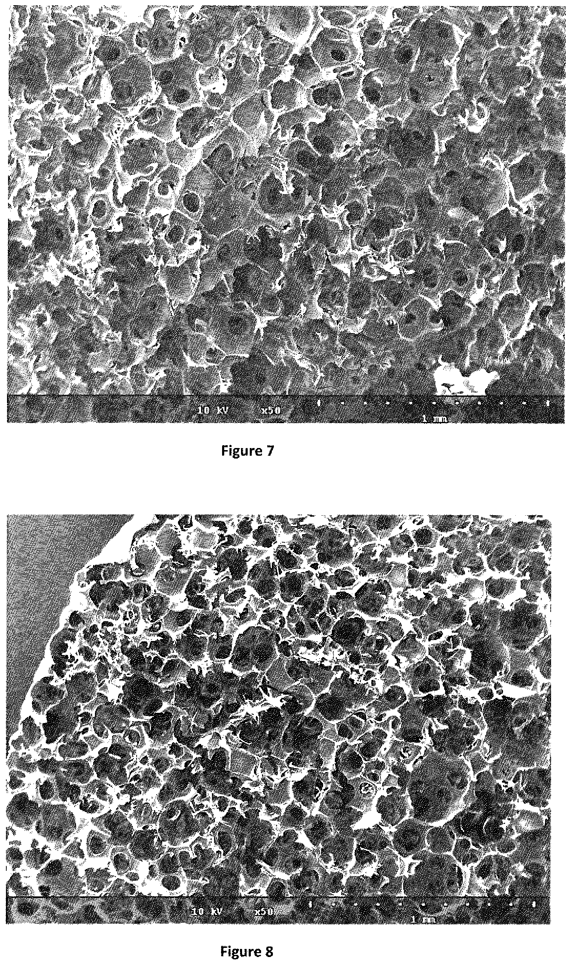

Les figures 6 à 8 montrent l'effet d'augmentation de la taille de ces interconnexions par infiltration d'un mélange acétone-éthanol à 30% en volume d'acétone, par rapport au volume total du mélange sur un empilement de microsphères d'agent porogène A qui sont en poly(méthacrylate de méthyle), après 15 min, 30 min et 1 heure d'infiltration.

Cette infiltration a lieu directement sur l'empilement de microsphères d'agent porogène A, avant l'introduction du verre bioactif M et/ou du polymère biodégradable P.

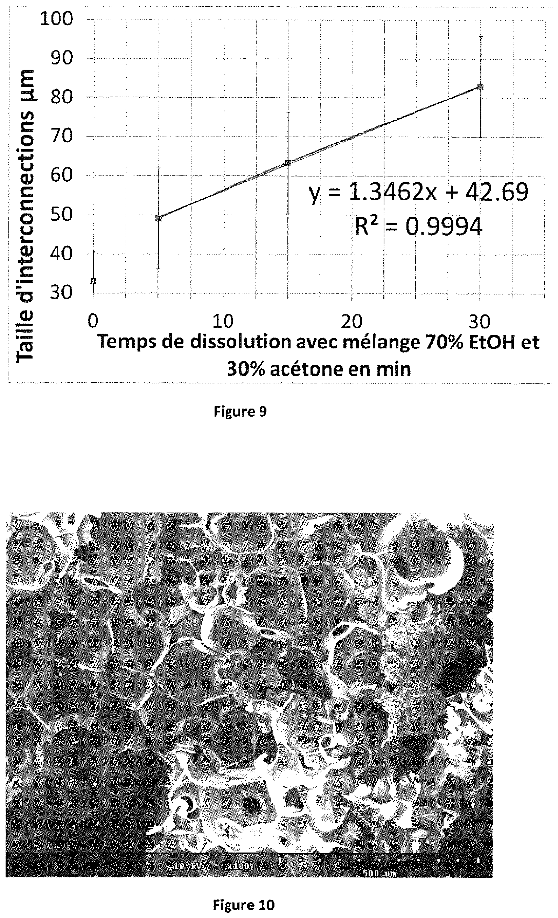

La figure 9 représente cet effet d'augmentation sous forme de courbe.

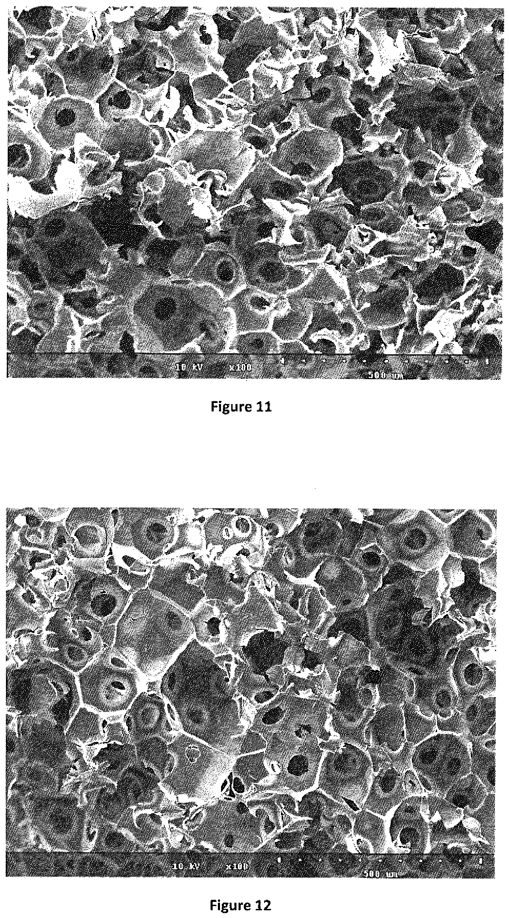

Les figures 10 à 12 montrent l'effet d'augmentation de la taille de ces interconnexions par chauffage à 125°C, de l'empilement de microsphères d'agent porogène A, avant l'introduction du verre bioactif M et/ou du polymère biodégradable P, pendant 15 min, 1 heure et 2 heures.

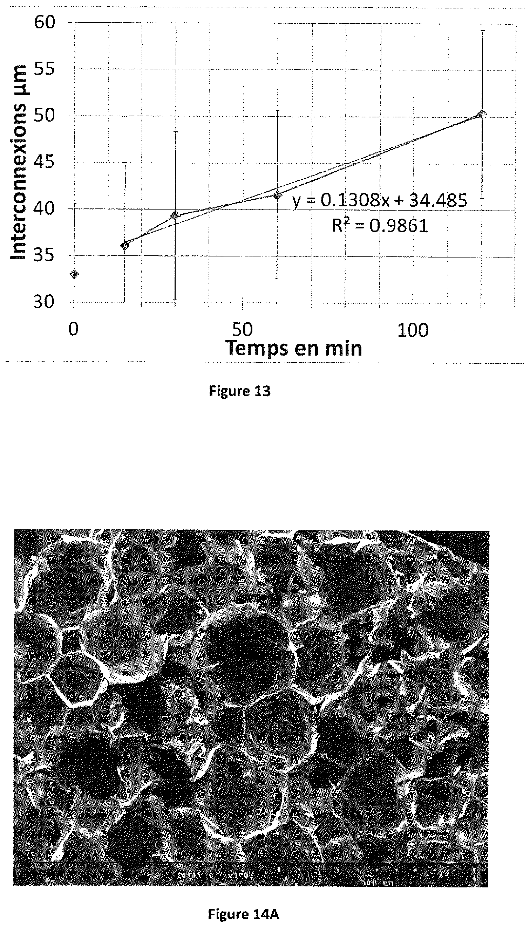

La figure 13 représente cet effet d'augmentation sous forme de courbe.

Afin de mieux faire comprendre l'invention, on va en décrire maintenant, à titre purement illustratif et non limitatif, plusieurs exemples de mise en oeuvre.

### Exemple 1: Fabrication d'un implant en un matériau composite comprenant, en tant que polymère biodégradable P, de la gélatine et un verre bioactif M constitué de 75% de SiO₂ et 25% de CaO, en masse, par rapport à la masse totale du verre, en tant que verre bioactif M (non partie de l'invention).

La gélatine a été choisie en tant que matériau pour le polymère biodégradable P car c'est un biopolymère naturel, biocompatible, bon marché et facilement disponible. La gélatine est de plus dérivée du collagène naturellement présent dans les os. Elle est d'autre part déjà utilisée dans le cadre d'applications cliniques, pansements, adhésifs et encapsulations de substances pharmaceutiques.

Un verre bioactif a été choisi en raison de sa grande capacité à induire la minéralisation, la possibilité de façonner leurs propriétés texturales et morphologiques (porosité, taille et donc surface spécifique) à l'échelle nanométrique, la grande gamme de compositions bioactives qu'il est possible de formuler, en y ajoutant par exemple des éléments anti-inflammatoires, ou ostéoinducteurs, et enfin c'est la combinaison de leurs propriétés de bioactivité et de biorésorbabilité qui en font les biomatériaux le plus prometteurs pour la régénération osseuse, notamment par rapport aux phosphates de calcium (céramique bioactive), qui sont en général soit moins bioactifs, soit moins résorbables.

Les microsphères sont des microsphères en polyméthacrylate de méthyle. Ce matériau a été choisi car il peut être aisément dissous par de nombreux solvants.

De plus, si des résidus de polyméthacrylate de méthyle non éliminés venaient à subsister dans le matériau d'implant, la bonne biocompatibilité de ce polymère avec les tissus humains est une bonne garantie que l'implant ne présentera aucun risque de cytotoxicité.

L'agent porogène était sous la forme de particules sphériques, à savoir des billes de polyméthacrylate de méthyle d'un diamètre compris entre 200 et 400µm. Il représentait 60% en volume du volume total du mélange (agent porogène A-polymère biodégradable P-verre bioactif M) introduit dans le moule.

Leurs diamètres peuvent être choisis entre plusieurs dizaines à plusieurs centaines de microns, suivant les applications. La porosité du matériau d'implant de l'invention qui sera finalement obtenu peut être contrôlée sur ces deux points ; premièrement le diamètre des pores qui sera obtenu dépend directement du diamètre des particules porogènes initiales. Il suffit donc d'ajuster la granulométrie des microsphères de polyméthacrylate de méthyle initiales en vue d'obtenir très simplement la porosité désirée. Deuxièmement la taille des interconnexions entre pores dépend directement de la taille de la zone de contact entre les billes polymères dans l'empilement initial. La taille de cette zone de contact peut être modifiée en faisant fusionner entre elles les particules de polymère initiales, au moyen d'un solvant S, ou par un traitement thermique préliminaire. Cette procédure a déjà été décrite par Descamps et al., "Manufacture of macroporous beta-tricalcium phosphate bioceramics". Journal of the European Ceramic Society 2008, 28, (1), 149-157 et "Synthesis of macroporous beta-tricalcium phosphate with controlled porous architectural". Ceramics International 2008, 34, (5), 1131-1137.

Dans cet exemple, le polymère biodégradable et le verre bioactif ont été utilisés pour obtenir une matrice composite.

Ainsi, à cet exemple, la première étape consistait à placer dans un moule à la taille et à la forme recherchées pour l'implant, des particules d'agents porogènes constituées de microsphères de polyméthacrylate de méthyle.

Dans une deuxième étape, on a introduit la poudre de verre bioactif.

La granulométrie de la poudre de verre bioactif joue un rôle important dans l'obtention d'une matrice composite homogène. De préférence, la granulométrie de la poudre de verre bioactif doit être très inférieure à 50 µm. Idéalement, la taille des particules de poudre doit être de l'ordre du micromètre, ou même de l'ordre du nanomètre à quelques centaines de nanomètres. Une telle finesse peut être obtenue au moyen d'un broyeur planétaire à billes par exemple.

Dans une troisième étape, la gélatine, préalablement dissoute dans de l'eau tiède, a été introduite dans le moule. Le mélange composite est alors homogénéisé.

Dans une quatrième étape, le mélange obtenu à la troisième étape est gélifié pendant plusieurs heures, dans le moule, la déshydratation partielle de la gélatine assurant la prise du mélange.

Cette opération est menée à une température comprise entre 0°C et 60°C, inclus, afin de ne pas dégrader la matrice.

Dans une cinquième étape, les microsphères d'agent porogène en polyméthacrylate de méthyle sont éliminées par lavage à l'acétone.

L'acétone présente plusieurs intérêts : tout d'abord les billes de polyméthacrylate de méthyle sont facilement dissoutes dans l'acétone et la gélatine est, quant à elle, insoluble dans l'acétone.

L'acétone permet de plus de poursuivre, si nécessaire, la déshydratation de la gélatine.

Enfin, c'est un solvant d'usage très courant, relativement économique, particulièrement disponible, qui ne représente pas de risques sérieux de toxicité.

Après plusieurs étapes de lavage, l'empreinte initiale des microsphères de polyméthacrylate de méthyle est complètement éliminée et le matériau final est obtenu, sous la forme d'un bloc macroporeux bio-composite en verre bioactif/gélatine.

La biodégradabilité de ce matériau d'implant en milieu vivant et sa tenue mécanique peuvent de plus être ajustées facilement en réticulant la gélatine lors d'une ultime étape d'immersion dans une solution d'un agent réticulant comme par exemple la génipine, le carbodiimide, le glutaraldéhyde, le formaldéhyde.

Cependant, cette étape est optionnelle.

Les structures obtenues peuvent être lavées sans aucun dommage dans des bains d'éthanol, afin d'éliminer d'éventuels résidus indésirables, tels que des chlorures, de l'acétone, etc.

A cet exemple, on a obtenu un matériau d'implant comprenant 60 % en masse de verre bioactif et 40 % en masse de gélatine, par rapport à la masse totale de l'implant et ayant 70%, en nombre de pores ayant au moins une interconnexion avec un autre pore.

### Exemple 2 : Fabrication d'un matériau d'implant selon l'invention avec une matrice en matériau hybride.

On a débuté par l'étape d'empilement des micro sphères d'agent porogène polyméthacrylate de méthyle dans un moule ayant la géométrie recherchée pour l'implant. Le volume des microsphères d'agent porogène A représentant 70% du volume total du mélange agent porogène A-polymère biodégradable P-précurseurs du verre bioactif M. Le matériau de l'agent porogène A était le poly(méthacrylate de méthyle). Les sphères avaient un diamètre compris entre 200 et 400 µm.

Dans une deuxième étape, le mélange hybride a été versé dans le moule contenant l'empilement de billes.

Une centrifugation ou une infiltration sous pression ou une infiltration sous vide peuvent être utilisées pour aider le mélange hybride à remplir les interstices entre les microsphères de poly(méthacrylate de méthyle).

Le matériau hybride a été obtenu par un procédé sol-gel.

Dans ce procédé, un sol contenant tous les précurseurs alcoxydes du verre bioactif est amené à gélifier par une succession de réactions de polymérisation.

Les précurseurs alcoxydes étaient en des quantités telles que la composition du verre bioactif était 75% SiO₂ et 25% CaO, en masse, par rapport à la masse total du verre bioactif obtenu au final.

Dans le cas du présent exemple, la gélatine (le polymère biodégradable P) a été ajoutée avant gélification du sol, de manière à obtenir un mélange hybride.

Pour la réalisation de matériau hybride, une difficulté majeure est que les traitements thermiques à haute et moyenne température, c'est-à-dire supérieurs à 150°C sont à proscrire.

Or, dans les procédés décrits dans l'art antérieur et notamment dans Lin, S. et al., "Nanostructure evolution and calcium distribution in sol-gel derived bioactive glass". Journal of Materials Chemistry 2009, 19, (9), 1276-1282, ces traitements thermiques sont indispensables pour l'obtention d'un réseau vitreux homogène, notamment pour l'incorporation du calcium au sein du réseau silicate.

L'utilisation d'un précurseur alcoxyde pour le calcium permet l'incorporation du calcium dans la phase inorganique sans traitement thermique.

Cependant la très grande réactivité des alcoxydes de calcium vis-à-vis des réactions d'hydrolyse/condensation en présence d'eau font que le sol obtenu est très instable, la polymérisation sol-gel ayant lieu extrêmement rapidement, ce qui a rendu impossible jusqu'à ce jour sa manipulation en vue de réaliser un implant poreux et n'a pas permis non plus une bonne incorporation du calcium dans le réseau silicate. Ainsi, les inventeurs ont découvert qu'en limitant au maximum l'introduction d'eau dans le sol et en utilisant un précurseur alcoxyde différent de celui utilisé dans la littérature (Ramila A. et al., "Synthesis routes for bioactive sol-gel glasses : alkoxides versus nitrates". Chemistry of Materials 2002, 14, (12), 542-548) (à savoir le méthoxyethoxyde de calcium), il est possible d'augmenter grandement la stabilité du sol. Les réactions d'hydrolyse/condensation sont alors suffisamment lentes pour permettre une incorporation homogène du calcium dans le réseau silicate, tout en restant suffisamment rapides pour permettre la polymérisation de la phase inorganique. Dans l'exemple, les précurseurs alcoxydes de silicium et calcium sont mélangés ensemble dans une solution alcoolique légèrement acidifiée. De préférence, les précurseurs alcoxydes sont le tétraéthoxysilane et l'éthoxyde de calcium. Ensuite la gélatine préalablement dissoute est ensuite ajoutée à ce mélange pour l'obtention d'un sol hybride. Les seuls apports d'eau se font par l'intermédiaire de l'acide et de la solution de gélatine : ceci est suffisant pour permettre les réactions d'hydrolyse/condensation tout en les limitant fortement de manière à avoir un sol stable et manipulable entre quelques minutes et quelques heures suivant les proportions des réactifs.

Le matériau d'implant de l'invention est alors obtenu en appliquant les quatrième et cinquième étapes telles que réalisées à l'exemple 1.

Que ce soit lors de l'élaboration du mélange composite ou du mélange hybride, il peut être avantageux d'ajouter un agent couplant, tel qu'un organo-alcoxysilane, au mélange.

En effet, deux classes d'implants hybrides organiques -inorganiques peuvent être réalisées, en fonction de la nature de l'interface qui associe les composantes organiques (polymère biocompatible) et inorganiques (verre bioactif). La classe I correspond à des systèmes hybrides dans lesquels les deux composantes interagissent par des liaisons faibles (liaisons hydrogène, Van der Waals, ou électrostatiques). Dans la classe II au contraire, les composantes organiques-inorganiques sont liées fortement par liaisons covalentes ou ionocovalentes. Ceci peut être obtenu au moyen d'un agent couplant.

Par exemple, l'agent couplant peut être simplement ajouté à la solution aqueuse du polymère biodégradable P, ici la gélatine. Le rôle de l'agent couplant est de fonctionnaliser la gélatine, en vue de permettre l'établissement de liaisons covalentes avec la phase inorganique (réseau silicate du verre bioactif). Dans le cas d'un mélange composite, le couplage permet d'obtenir des particules de verre bioactif liées en surface à la gélatine. Dans le cas d'un mélange hybride, un véritable copolymère organo-minéral (hybride de classe II) est obtenu. L'intérêt est de pouvoir maîtriser à façon la dégradabilité de l'implant composite ou hybride ainsi que sa tenue mécanique, en agissant simplement sur le degré d'affinité entre phases organique et inorganique.

Un exemple d'agent couplant utilisé avec succès dans l'invention est le GPTMS (3-glycidoxypropyltriméthoxysilane), qui est soluble dans une solution aqueuse de gélatine.

On a obtenu un matériau d'implant constitué de 70% en masse de gélatine et de 30% en masse de verre bioactif.

### Exemple 3 : Fabrication d'un matériau d'implant à matrice polymère biodégradable P recouverte de verre bioactif (non partie de l'invention).

On a procédé comme à l'exemple 2, sauf qu'à la deuxième étape, seule la gélatine a été introduite, et qu'après la cinquième étape l'élimination par lavage des microsphères de polyméthacrylate de méthyle, le polymère biodégradable P, ici la gélatine a été réticulée dans une solution de glutaraldéhyde.

La quantité d'agent porogène A était de 70% en volume par rapport au volume total agent porogène A-polymère biodégradable P introduit dans le moule.

Ensuite, la matrice en polymère biodégradable P est immergée dans une suspension du verre bioactif M ou encore immergée dans un sol contenant tous les précurseurs alcoxydes du verre bioactif M.

Dans les deux cas, la matrice 1 est ensuite séchée pour permettre le dépôt des particules de verre bioactif M ou la gélification du sol selon le cas.

### Exemple 4 : Fabrication d'un matériau hybride par le procédé de l'invention.

### Produits utilisés :

- Téraéthylorthosilicate TEOS
- Ethoxyde de Calcium Ca(OEt)₂
- 3-glycidoxypropyltrimethoxysilane GPTMS
- HCl 2M et HCl 10mM
- Ethanol absolu
- Gélatine type B
- Acétone

### Protocole :

1. Remplir un tube en polyéthylène de 32 mm de hauteur et 9 mm de diamètre avec des billes de PMMA sur une hauteur d'environ 10 mm.
2. Mélanger 7,80g de TEOS et 6,39g d'éthanol dans un flacon.
3. Agiter pendant 15 min à l'aide d'un agitateur magnétique.
4. Ajouter 1,35mL de HCl à 2M dans le mélange Ethanol + TEOS.
5. Agiter pendant 30 min.
6. Peser 6,39 g d'éthanol dans un autre flacon.
7. Ajouter 1,74g d'éthoxyde de calcium.
8. Agiter 15 min.
9. Ajouter le sol contenant le TEOS à la solution d'éthoxyde de calcium.
10. Agiter au moins 1 heure.
11. Dissoudre 1,26g de gélatine de type B et 0,63g de GPTMS dans 8,74g d'HCl 10mM dans un bain marie à 60°C.
12. Prélever 3g de sol de bioverre et ajouter 7g de sol de gélatine greffé GPTMS dans un flacon.
13. Agiter quelques minutes, avec un agitateur magnétique.
14. Ajouter le sol hybride sur les billes PMMA.
15. Centrifuger 1 min
16. Laisser gélifier à une température comprise entre 0°C et 60°C, au moins 24 heures
17. Démouler le bloc hybride obtenu.
18. Dissolution des billes PMMA dans un flacon rempli d'acétone en renouvelant l'acétone au bout de 24 heures. Cette opération est à réitérer 2 fois.
19. Récupérer le bloc poreux obtenu et le mettre à sécher à l'étuve à 60°C pendant 24 heures

On a obtenu un matériau d'implant constitué d'un matériau hybride de classe II composé de 70% en masse de polymère biodégradable P et de 30% en masse de verre bioactif M.

### Exemple 5 : Préparation d'implants poreux composites avec 60% de bioverre (75 % SiO₂- 25% CaO) et 40% de gélatine (% massiques) (non partie de l'invention)

### 1) Synthèse de la poudre de verre par voie sol-gel

13,48 mL d'eau et 13,48 mL d'éthanol sont mélangés à 2,25 mL d'HCl à 2N puis 13,94 mL de TEOS sont ajoutés. Après 30 minutes d'agitation 5,2637 g de Ca(NO₃)₂.4H₂O sont ajoutés. Le sol est agité pendant 1 heure, mis à l'étude à 60°C dans des conteneurs en téflon pendant 24h puis mis à l'air à 125°C durant 24h. La poudre ainsi obtenue est ensuite calcinée pendant 24 heures à 700°C (chauffage de 25 à 700°C effectué en 2 heures).

La poudre est alors broyée pendant 30 minutes puis tamisée pour ne conserver que la fraction inférieure à 50 µm.

### 2) Préparation du composite

De la poudre de gélatine de porc (type A) est ajoutée à de l'eau distillée chauffée à 35°C selon un rapport de 0,1 g/mL d'eau, le mélange est agité pendant 10 minutes. Parallèlement, une quantité de 0,025 g de poudre de verre est mélangée à 0,2 g de billes PMMA de diamètres compris entre 100 et 300µm et représentant 60% en volume, du volume total du mélange introduit dans le moule. 0,15 mL de solution de gélatine dans l'eau sont alors ajoutés, le mélange obtenu est versé dans un tube dans lequel il est compacté.

Après 1 jour de séchage à l'air ambiant, le cylindre de verre + billes + gélatine est sorti du moule et immergé dans de l'acétone pendant 6 heures sous agitation, l'acétone est alors renouvelé et la dissolution est laissée se poursuivre 24 heures, toujours sous agitation. Le bloc composite poreux verre-gélatine obtenu est alors rincé à l'acétone et séché à l'air ambiant. Il est constitué de 60% en masse de verre bioactif et 40% en masse de polymère biodégradable.

### Exemple 6 : Evaluation in vitro des implants obtenus aux exemples 1 à 5.

La bioactivité des matériaux d'implants obtenus aux exemples 1 à 3 a été évaluée *in vitro* en les immergeant dans une solution physiologique (SBF) ayant une composition ionique identique à celle du plasma sanguin (test ISO-23317).

On a alors vérifié la grande bioactivité caractéristique des verres bioactifs utilisés dans les matériaux d'implants : ces matériaux d'implants se révélant très prompts à induire la minéralisation au contact du milieu physiologique : dès 1 h d'interaction avec le milieu, une partie des ions calcium issus de la matrice vitreuse a migré en surface du composite, où des ions phosphates provenant du milieu physiologique ont été incorporés pour former une couche de phosphate de calcium d'une dizaine de microns d'épaisseur, qui revêt la surface des pores.

Ceci constitue la première étape du processus de bioactivité.

On a vérifié que par la suite cette couche de phosphate de calcium continue à croître pour former une couche apatitique analogue au minéral osseux.

La réticulation de la gélatine n'amoindrit pas la bioactivité de l'implant, mais permet d'augmenter sa résistance à la dissolution en milieu physiologique.

On a également noté que le milieu SBF est rapidement (dès 1 jour) épuisé en phosphore, et dans une moindre mesure en calcium, ces éléments étant incorporés en surface des implants et donc retranchés du milieu pour former une couche de phosphate de calcium biomimétique.

La réticulation de la gélatine n'altère aucunement la réactivité chimique des implants, mais présente l'avantage de permettre d'ajuster leur biodégradabilité en milieu vivant.

Ainsi, tous les matériaux fabriqués aux exemples 1 à 5 se révèlent prompts à induire la formation de minéral osseux au contact des fluides physiologiques.

Cependant, on note des différences entre ces matériaux.

Tout d'abord, les matériaux dans lesquels la gélatine a été réticulée ont une biodégradabilité ralentie, ce qui se manifeste par la mise en solution plus lente du silicium. La formation de phosphates de calcium est également ralentie.

Ensuite, on constate que la formation de phosphates de calcium à la surface du matériau est plus lente avec le matériau composite qu'avec le matériau constitué du polymère biodégradable P revêtu du verre bioactif M et qu'avec le matériau hybride de l'invention.

Avec les matériaux composite et polymère biodégradable revêtu du verre bioactif comme attendu, la formation des phosphates de calcium et en particulier d'apatite, ne se fait qu'en surface du matériau.

En contraste, et de façon surprenante, avec le matériau hybride, la formation de phosphates de calcium se fait non seulement en surface mais également dans la masse, ce qui représente un avantage certain, en particulier lorsque le défaut osseux à combler nécessite une intégration rapide.

### Exemple comparatif : Fabrication d'un implant selon WO 2013/023064.

De la poudre de gélatine de porc (type A) est ajoutée à de l'eau distillée chauffée à 35°C selon un rapport de 0,1 g/mL d'eau, le mélange est agité pendant 10 minutes. Une quantité de 0,75 g de poudre de verre est mélangée à 57,38 g de particules de NaCl, puis 4,5 mL de la solution de gélatine sont alors ajoutés, de sorte à ce que le volume de porogène représente 90 % du volume total du mélange, comme indiqué dans WO 2013/023064. Le mélange obtenu est versé dans un tube. Le moule et son mélange subissent alors une congélation, puis une lyophilisation sous vide pendant une journée. Après lyophilisation, le bloc composite est démoulé et immergé dans de l'eau distillée afin de dissoudre le porogène (NaCl).

Malheureusement, la quantité de porogène (90% du volume total) s'est avérée beaucoup trop importante relativement à la quantité de mélange composite : la dissolution du porogène a entraîné la destruction immédiate de la structure composite, et aucun implant n'a pu être obtenu par ce protocole d'élaboration.

### Exemple 7 : Fabrication d'un matériau d'implant à matrice en matériau hybride de classe II dans lequel le polymère biodégradable P est du poly(acide D,L-lactique).

On a procédé comme à l'exemple 2, sauf que la gélatine a été remplacée par un poly(acide D,L-lactique) (PDLLA) de classe II.

La composition du verre bioactif était de 75% de SiO₂ et 25% de CaO, en masse, par rapport à la masse totale du verre et le matériau d'implant final avait la composition 30% verre bioactif - 70% PDLLA, en masse, par rapport à la masse total du matériau d'implant obtenu.

L'agent porogène A était des particules sphériques de paraffine d'un diamètre compris entre 400 et 600 µm.

Les microsphères représentaient 70% en volume du volume total du mélange introduit dans le moule.

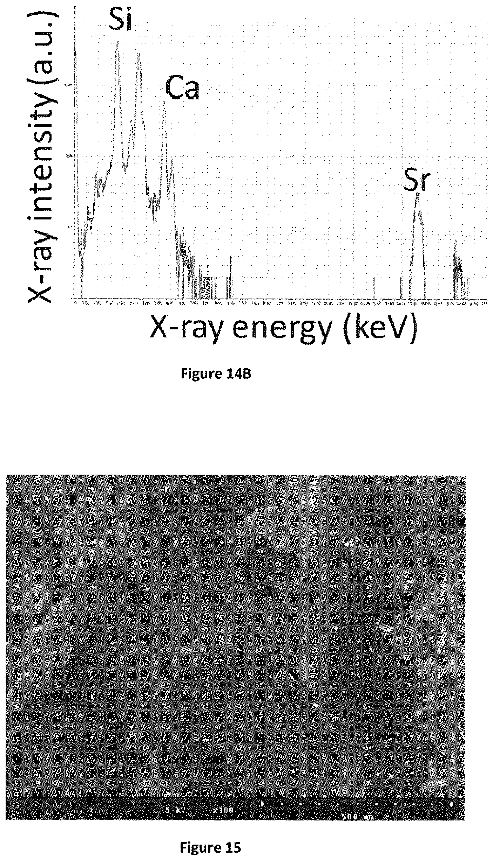

Comme on peut le voir en figure 15, le matériau d'implant obtenu a plus de 70% en nombre de pores sphériques et interconnectés.

Le solvant S1 était l'acétone.

Le solvant S était le cyclohexane.

### Exemple 8 : Fabrication d'un matériau d'implant selon l'invention à matrice en matériau hybride de classe II dans lequel le polymère biodégradable P est du chitosan.

On a procédé comme à l'exemple 2, sauf que la gélatine a été remplacée par du chitosan.

Le verre bioactif utilisé avait la composition massique 75% SiO₂ - 25% CaO et le matériau d'implant obtenu avait la composition 30% verre bioactif - 70% chitosan, en masse, par rapport à la masse totale du matériau d'implant.

L'agent porogène A était des particules sphériques de poly(méthacrylate de méthyle) (PMMA) d'un diamètre de 200µm. Il représentait 70% en volume du volume total du mélange introduit dans le moule.

Le solvant S1 était une solution aqueuse acide (pH = 2).

Le solvant S était l'acétone.

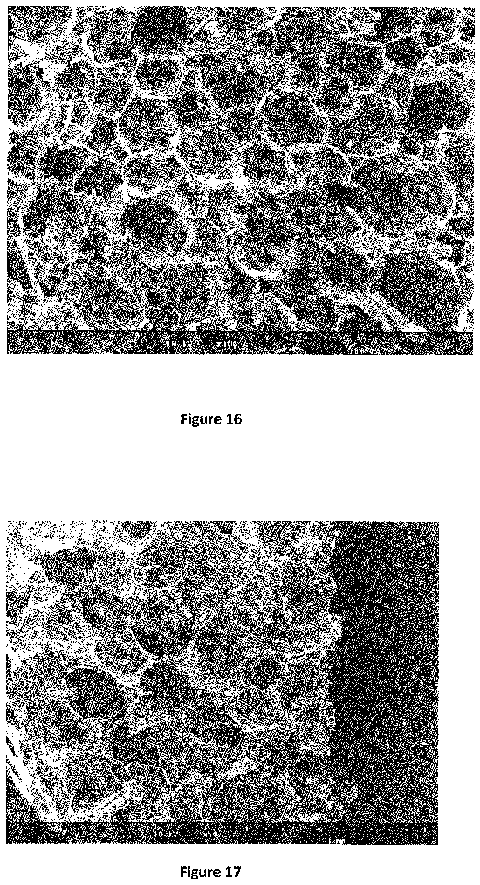

Comme on peut le voir en figure 16, ce matériau d'implant a des pores sphériques ayant tous au moins une interconnexion avec au moins un autre pore.

Dans ce matériau d'implant, plus de 90% en nombre des pores sont sphériques.

**Exemple 9 :** Fabrication d'un matériau d'implant selon l'invention à matrice en matériau hybride de classe II dans lequel le polymère biodégradable P est du polyéthylène glycol de poids moléculaire moyen en masse égal à 35 000.

On a procédé comme à l'exemple 2, sauf que l'on a remplacé la gélatine par du polyéthylène glycol de poids moléculaire moyen en masse de 35 000.

Le verre bioactif avait la composition 75% SiO₂ - 25% CaO, en masse, par rapport à la masse totale du verre bioactif obtenu en final et le matériau d'implant obtenu avait la composition 30% verre bioactif - 70% PEG, par rapport à la masse totale du matériau d'implant.

L'agent porogène A était des particules sphériques de paraffine d'un diamètre compris entre 200 et 400 µm. Ces particules représentaient 70% en volume du volume total du mélange introduit dans le moule.

Le solvant S1 était de l'éthanol chauffé à 60°C.

Le solvant S était du cyclohexane.

Comme on peut le voir en figure 17, on obtient un matériau d'implant ayant plus de 90% en nombre de pores sphériques ayant au moins une interconnexion avec au moins un autre pore.

### Exemple 10 : Fabrication d'un matériau d'implant selon l'invention à matrice en matériau hybride de classe II dans lequel le polymère biodégradable P est du poly(alcool vinylique) (PVA).

On a procédé comme à l'exemple 2, sauf que l'on a remplacé la gélatine par du poly(alcool vinylique) .

L'agent porogène A était des microsphères de PMMA d'un diamètre compris entre 100 et 300µm. Ces microsphères représentaient 70% en volume du volume total du mélange introduit dans le moule.

Le solvant S1 était l'eau et chauffée à 80°C.

Le solvant S était l'acétone.

La composition du verre bioactif était 75% SiO₂ - 25% CaO, en masse, par rapport à la masse totale du verre bioactif, et la composition du matériau d'implant final obtenu était 30% verre bioactif- 70% poly(alcool vinylique).

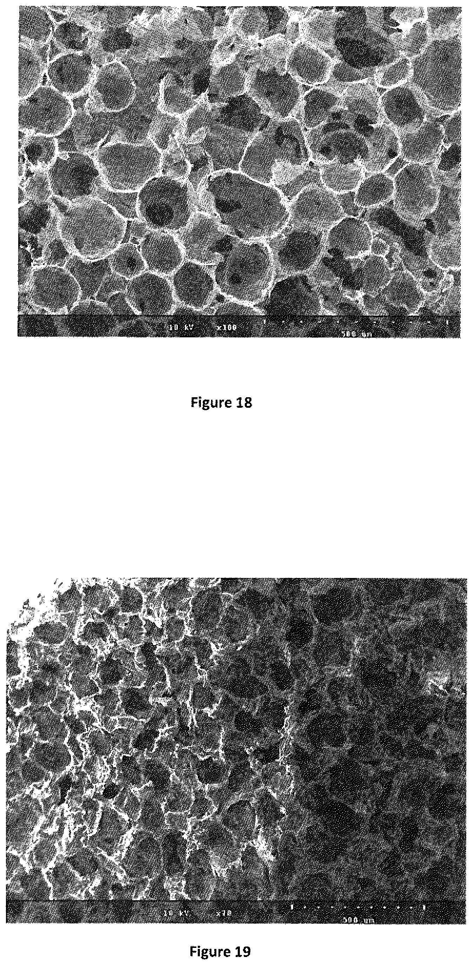

Comme on peut le voir en figure 18, ce matériau d'implant a plus de 80% en nombre de pores sphériques ayant de plus, tous au moins une interconnexion avec au moins un autre pore.

### Exemple 11 : Fabrication d'un matériau d'implant selon l'invention à matrice en matériau hybride de classe I dans lequel le polymère biodégradable P est du carraghénane.

On a procédé comme à l'exemple 2, sauf que l'on a remplacé la gélatine par du carraghénane.

La composition du verre bioactif était 75% SiO₂ - 25% CaO, en masse, par rapport à la masse totale du verre bioactif, et la composition finale de l'implant obtenu était 30% verre bioactif - 70% carraghénane, en masse, par rapport à la masse totale du matériau d'implant.

L'agent porogène A était des microsphères de PMMA d'un diamètre compris entre 100 et 300µm. Elles représentaient 60% en volume du volume total du mélange introduit dans le moule.

Le solvant S1 était l'eau chauffée à 80°C.

Le solvant S était l'acétone.

Comme on peut le voir en figure 19, ce matériau d'implant a plus de 70% en nombre de pores sphériques, tous interconnectés avec au moins un autre pore.

### Exemple 12 : Fabrication d'un matériau d'implant selon l'invention à matrice en matériau hybride de classe I dans lequel le polymère biodégradable P est du collagène.

On a procédé comme à l'exemple 2, sauf que l'on a remplacé la gélatine par du collagène.

Le verre bioactif avait la composition 75% SiO₂ - 25% CaO, en masse, par rapport à la masse totale du verre bioactif, et le matériau d'implant obtenu avait la composition 30% verre bioactif - 70% collagène, en masse, par rapport à la masse totale du matériau d'implant obtenu.

L'agent porogène A était des microsphères de PMMA d'un diamètre compris entre 100 et 300µm. Elles représentaient 70% en volume du volume total du mélange introduit dans le moule.

Le solvant S1 était une solution aqueuse à pH acide, de préférence à pH = 2.

Le solvant S était l'acétone.

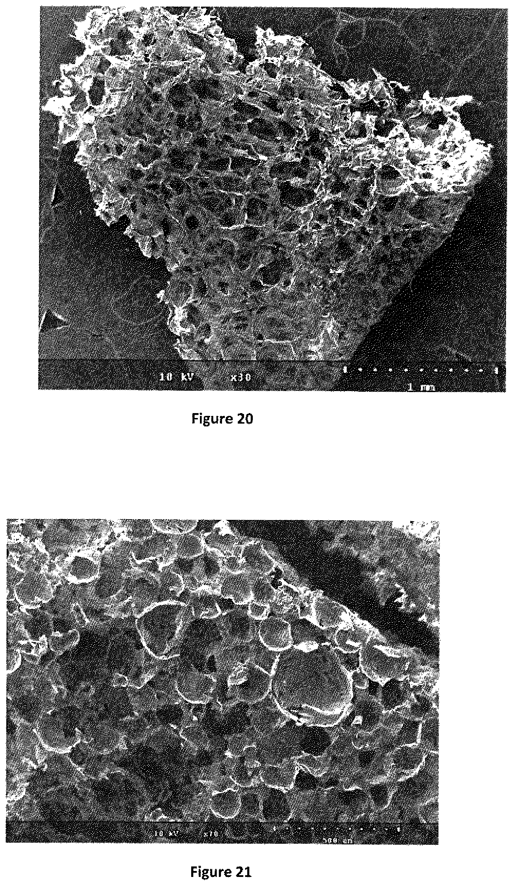

Comme on le voit en figure 20, le matériau d'implant obtenu a plus de 70% en nombre de pores sphériques, ayant tous au moins une interconnexion avec au moins un autre pore.

### Exemple 13 : Fabrication d'un matériau d'implant selon l'invention à matrice en matériau hybride de classe I dans lequel le polymère biodégradable P est de l'acide hyaluronique.

On a procédé comme à l'exemple 2, sauf que l'on a remplacé la gélatine par de l'acide hyaluronique.

La composition du verre bioactif était 75% SiO₂ - 25% CaO, en masse, par rapport à la masse totale du verre bioactif, et la composition du matériau d'implant était 30% verre bioactif - 70% acide hyaluronique, en masse, par rapport à la masse totale du matériau d'implant obtenu.

L'agent porogène A était des microsphères de PMMA d'un diamètre compris entre 100 et 300µm. Elles représentaient 60% en volume du volume total du mélange introduit dans le moule.

Le solvant S1 était une solution aqueuse acide (pH = 2).

Le solvant S était l'acétone.

Comme on le voit en figure 21, le matériau d'implant obtenu a plus de 70% en nombre de pores sphériques, ayant tous au moins une interconnexion avec au moins un autre pore.

### Exemple 14 : Fabrication d'un matériau d'implant selon l'invention à matrice en matériau hybride de classe I dans lequel le polymère biodégradable P est du poly(caprolactone) .

On a procédé comme à l'exemple 2, sauf que l'on a remplacé la gélatine par du poly(caprolactone).

La composition du verre bioactif était 75% SiO₂ - 25% CaO, en masse, par rapport à la masse totale du verre bioactif, et le matériau d'implant obtenu avait la composition 30% verre bioactif - 70% poly(caprolactone), en masse, par rapport à la masse totale du matériau d'implant.

L'agent porogène A était des billes de paraffine d'un diamètre compris entre 200 et 400µm. Elles représentaient 70% en volume du volume total du mélange introduit dans le moule.

Le solvant S1 était le chloroforme.

Le solvant S était le cyclohexane.

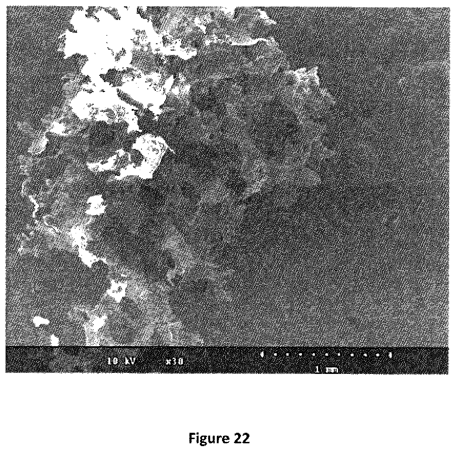

Comme on le voit en figure 22, le matériau d'implant obtenu a plus de 70 % en nombre de pores sphériques.

Comme on peut également le voir sur la figure 22, au moins 70% en nombre de ces pores ont au moins une interconnexion avec au moins un autre pore.

### Exemple 15 : Fabrication d'un matériau d'implant selon l'invention à matrice en matériau hybride de classe I dans lequel le polymère biodégradable P est du dextran.

On a procédé comme à l'exemple 2, sauf que l'on a utilisé du dextran à la place de la gélatine.

Le matériau d'implant obtenu a plus de 90% en nombre de pores sphériques ayant tous au moins une interconnexion avec au moins un autre pore.

La composition du verre bioactif était 75% SiO₂ - 25% CaO, en masse.

L'agent porogène A était des billes de PMMA d'un diamètre compris entre 100 et 300µm. Elles représentaient 70% en volume du volume total du mélange introduit dans le moule.

Le solvant S1 était une solution aqueuse acide (pH = 2).

Le solvant S était l'acétone.

Le matériau d'implant avait la composition 70% en masse dextran-30% en masse verre bioactif.

### Exemple 16 : Fabrication d'un matériau d'implant selon l'invention à matrice en matériau hybride dans lequel le verre bioactif est un verre dopé au strontium.

On a procédé comme à l'exemple 2 sauf que l'on a ajouté un alcoxyde de strontium (par exemple l'isopropoxyde de strontium) aux précurseurs de silicium et de calcium pour obtenir un verre bioactif ayant la composition massique 75% SiO₂-20% CaO - 5% SrO.

Comme on peut le voir en figure 14A, le matériau d'implant obtenu a plus de 70% en nombre de pores sphériques ayant au moins une interconnexion avec un autre pore.

Il est composé d'un matériau hybride de classe II dans lequel le verre bioactif a la composition massique 75% SiO₂-20% CaO - 5% SrO comme on peut le voir en figure 14B, et représente 30% en masse de la masse totale du matériau, les 70% en masse restants étant la gélatine.

### Caractérisation de la sphéricité des macropores obtenus

La voie de synthèse proposée permet l'obtention de pores sphériques. En effet, en mesurant deux diamètres perpendiculaires pour chaque pore, le rapport du plus petit diamètre sur le plus grand diamètre est en moyenne de **0,9 ± 0,1.**

Ainsi, il apparaît clairement que grâce aux procédés de l'invention, des implants ayant toutes les propriétés de porosité, en termes de tailles de pores, sphéricité de ces pores, distribution de cette taille des pores dans une très large gamme comprise entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant, peuvent être obtenus, en association avec des interconnexions entre pores dont la plus petite dimension est comprise entre 25 microns et 250 micromètres inclus, ce qui n'avait jamais été obtenu auparavant.

## Revendications

1. Procédé de fabrication d'un implant en un matériau hybride pour le comblement de défauts osseux, la régénération osseuse et l'ingénierie tissulaire de l'os, **caractérisé en ce qu'**il comprend les étapes suivantes :
a) sélection d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S différent du solvant S1,
c) sélection de microsphères d'un agent porogène A ayant des diamètres et des tailles correspondant aux diamètres et tailles recherchés des pores dans le matériau constituant l'implant à fabriquer, cet agent porogène A étant :
- en un polymère insoluble dans le au moins un solvant S1 et soluble dans le au moins un solvant S,
le au moins un solvant S dans lequel le matériau du polymère biodégradable P est insoluble et le au moins un solvant S dans lequel le matériau de l'agent porogène A est soluble étant identiques,
d) introduction des microsphères de l'agent porogène A dans un moule ayant la forme et la taille recherchées pour l'implant, ces microsphères formant un empilement compact correspondant à la forme et à la taille des pores à obtenir dans le matériau d'implant, et représentant au moins 60% en volume, de préférence 70% en volume par rapport au volume total du mélange agent porogène A-polymère biodégradable P-précurseurs alcoxydes du verre bioactif M,
e) introduction du polymère biodégradable P dans les précurseurs alcoxydes du verre bioactif M,
f) introduction du mélange obtenu à l'étape e) dans le moule,
g) gélification du mélange contenu dans le moule après l'étape f),
h) démoulage du mélange obtenu à l'étape g),
i) élimination des microsphères d'agent porogène A par lavage avec le solvant S.

2. Procédé selon la revendication 1, **caractérisé en ce que** l'étape e) et/ou l'étape f) sont mises en oeuvre avant l'étape d).

3. Procédé selon la revendication 1, **caractérisé en ce que** les étapes d) et e) et f) sont mises en oeuvres simultanément.

4. Procédé selon l'une quelconque des revendications 1 à 3, **caractérisé en ce qu'**il comprend de plus une étape j) de réticulation du matériau obtenu à l'étape i).

5. Procédé selon l'une quelconque des revendications 1 à 4, **caractérisé en ce que** le polymère biodégradable P est choisi parmi :
- les polymères biodégradables solubles dans au moins un solvant S1 et insolubles dans au moins un solvant S choisis parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine, ,
- les polyesters biorésorbables de préférence l'alcool polyvinylique ou le poly(acide lactique),
- les polymères synthétiques biodégradables, de préférence un polyéthylène glycol, ou le poly(caprolactone),
- les protéines, de préférence la gélatine ou le collagène,
et **en ce que** le matériau de l'agent porogène A est choisi parmi les polymères biodégradables insolubles dans le au moins un solvant S1 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène, ou l'acrylonitrile butadiène styrène (ABS).
le matériau de l'agent porogène A étant différent du polymère biodégradable P.

6. Procédé selon l'une quelconque des revendications 1 à 5, **caractérisé en ce que** le rapport en poids polymère biodégradable P/ verre bioactif M est compris entre 20/80 et 80/20, bornes incluses.

7. Procédé selon l'une quelconque des revendications 1 à 6, **caractérisé en ce que** le verre bioactif M est un verre à base de SiO₂ et de CaO, le polymère biodégradable P est la gélatine, le matériau des microsphères d'agent porogène A est le polyméthacrylate de méthyle et le solvant S est l'acétone.

8. Procédé selon l'une quelconque des revendications 1 à 7, **caractérisé en ce qu'**il comprend de plus, à l'étape f), une étape d'introduction d'un agent de couplage, de préférence un composé organoalkoxysilane, plus préférablement du 3-glycidoxypropyltriméthoxysilane (GPTMS), encore plus préférablement du 3-glycidoxypropyltriéthoxysilane (GPTES).

9. Procédé selon l'une quelconque des revendications 1 à 8, **caractérisé en ce qu'**il comprend après l'étape d), et avant l'étape e), une étape d'agrandissement des interconnexions (4), par infiltration d'un solvant S du matériau de l'agent porogène A, dans l'empilement des microsphères d'agent porogène A et/ou par chauffage de cet empilement.

10. Procédé de fabrication d'un matériau hybride comprenant un polymère biodégradable P et un verre bioactif M à base de SiO₂ et de CaO et contenant optionnellement du P₂O₅ et/ou optionnellement dopé au strontium **caractérisé en ce qu'**il comprend les étapes suivantes :
A) préparation d'un sol des précurseurs alcoxydes d'un verre bioactif M à base de SiO₂ et de CaO, contenant optionnellement P₂O₅ et/ou optionnellement dopé au strontium,
B) ajout d'une solution contenant le polymère biodégradable P, dissout dans un solvant S, dans le sol de l'étape A),
C) polymérisation sol-gel des précurseurs alcoxydes et gélification du mélange obtenu à l'étape B) à une température comprise entre 0°C et 60°C, sous air.

11. Matériau d'implant obtenu par le procédé décrit dans les revendications 1 à 9, pour le comblement de défauts osseux, la régénération osseuse et l'ingénierie tissulaire de l'os,
**caractérisé en ce qu'il comprend un matériau hybride comprenant :**
• un verre bioactif M à base de SiO₂ et CaO, optionnellement contenant P₂O₅ et/ou optionnellement dopé au strontium, et
• un polymère biodégradable P soluble dans au moins un solvant S1 choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, le poly(caprolactone), et
- les protéines, de préférence la gélatine ou le collagène,
**et en ce que** il consiste en une matrice (1) comprenant le matériau hybride, cette matrice ayant au moins 70 % en nombre de pores (2) ayant la forme de sphères ou de polyèdres s'inscrivant dans une sphère, le diamètre (3) des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions (4) entre les pores ayant leur plus petite dimension comprise entre 25 µm et 250 µm, bornes incluses, au moins 70% en nombre de pores (2) ayant au moins une interconnexion avec au moins un autre pore.

12. Implant en un matériau hybride pour le comblement de défauts osseux, la régénération osseuse et l'ingénierie tissulaire de l'os, **caractérisé en ce qu'**il comprend un matériau selon la revendication 11.

## Patentansprüche

1. Verfahren zur Herstellung eines Implantats aus einem Hybridmaterial zum Auffüllen von Knochendefekten, zur Knochenregeneration und zur Knochengewebezüchtung, **dadurch gekennzeichnet, dass** es die folgenden Schritte umfasst:
a) Auswahl eines bioaktiven Glases M auf Basis von SiO₂ und CaO, optional P₂O₅ enthaltend und/oder optional mit Strontium dotiert,
b) Auswahl eines biologisch abbaubaren Polymers P, das in mindestens einem Lösungsmittel S1 löslich ist und in mindestens einem Lösungsmittel S, das sich von dem Lösungsmittel S1 unterscheidet, unlöslich ist,
c) Auswahl von Mikrosphären eines porenbildenden Mittels A, welche Durchmesser und Größen aufweisen, die den angestrebten Durchmessern und Größen der Poren in dem Material entsprechen, welches das herzustellende Implantat bildet, wobei das porenbildende Mittel A:
- aus einem Polymer besteht, das in dem mindestens einen Lösungsmittel S1 unlöslich ist, und in dem mindestens einen Lösungsmittel S löslich ist,
wobei das mindestens eine Lösungsmittel S, in dem das Material des biologisch abbaubaren Polymers P unlöslich ist, und das mindestens eine Lösungsmittel S, in dem das Material des porenbildenden Mittels A löslich ist, identisch sind,
d) Einbringen der Mikrosphären des porenbildenden Mittels A in eine Gießform, die die für das Implantat gesuchte Form und Größe aufweist, wobei diese Mikrosphären eine kompakte Stapelung bilden, die der Form und der Größe der Poren entspricht, die in dem Implantat-Material erhalten werden sollen, und mindestens 60 Volumen-%, vorzugsweise 70 Volumen-% bezogen auf das Gesamtvolumen des Gemisches an porenbildendem Mittel A-biologisch abbaubarem Polymer P-Alkoxid-Vorläufern des bioaktiven Glases M repräsentieren,
e) Einbringen des biologisch abbaubaren Polymers P in die Alkoxid-Vorläufer des bioaktiven Glases M,
f) Einbringen des in Schritt e) erhaltenen Gemisches in die Gießform,
g) Gelieren des in der Gießform enthaltenen Gemisches nach dem Schritt f),
h) Entformen des in Schritt g) erhaltenen Gemisches,
i) Eliminieren der Mikrosphären des porenbildenden Mittels A durch Waschen mit dem Lösungsmittel S.

2. Verfahren nach Anspruch 1, **dadurch gekennzeichnet, dass** der Schritt e) und/ oder der Schritt f) vor dem Schritt d) durchgeführt werden.

3. Verfahren nach Anspruch 1, **dadurch gekennzeichnet, dass** die Schritte d) und e) und f) zeitgleich durchgeführt werden.

4. Verfahren nach einem der Ansprüche 1 bis 3, **dadurch gekennzeichnet, dass** es zusätzlich einen Schritt j) des Vernetzens des in Schritt i) erhaltenen Materials umfasst.

5. Verfahren nach einem der Ansprüche 1 bis 4, **dadurch gekennzeichnet, dass** das biologisch abbaubare Polymer P ausgewählt wird aus:
- biologisch abbaubaren Polymeren, die in mindestens einem Lösungsmittel S1 löslich und in mindestens einem Lösungsmittel S unlöslich sind, ausgewählt aus:
- biologisch resorbierbaren Polysacchariden, vorzugsweise ausgewählt aus Dextran, Hyaluronsäure, Agar, Chitosan, Alginsäure, Natrium- oder Kaliumalginat, Galaktomannan, Carrageen, Pektin,
- biologisch resorbierbaren Polyestern, vorzugsweise Polyvinylalkohol oder Poly(Milchsäure),
- biologisch abbaubaren synthetischen Polymeren, vorzugsweise einem Polyethylenglycol oder Poly(Caprolacton),
- Proteinen, vorzugsweise Gelatine oder Kollagen,
und dadurch, dass das Material des porenbildenden Mittels A ausgewählt wird aus den biologisch abbaubaren Polymeren, die in dem mindestens einen Lösungsmittel S1 unlöslich und in dem mindestens einen Lösungsmittel S löslich sind, vorzugsweise ausgewählt aus C₁- bis C₄-Polyalkylmethacrylaten, vorzugsweise Polymethylmethacrylat oder Polybutylmethacrylat, Polyurethan, Polyglycolsäure, verschiedenen Formen von Polymilchsäuren, Copolymeren von Milch-Co-Glycolsäuren, Polycaprolacton, Polypropylenfumarat, Paraffin und Naphthalin oder Acrylnitril-Butadien-Styrol (ABS),
wobei sich das Material des porenbildenden Mittels A von dem biologisch abbaubaren Polymer P unterscheidet.

6. Verfahren nach einem der Ansprüche 1 bis 5, **dadurch gekennzeichnet, dass** das Gewichtsverhältnis biologisch abbaubares Polymer P/bioaktives Glas M im Bereich zwischen 20/80 und 80/20 enthalten ist, einschließlich der Extreme.

7. Verfahren nach einem der Ansprüche 1 bis 6, **dadurch gekennzeichnet, dass** das bioaktive Glas M ein Glas auf Basis von SiO₂ und CaO ist, das biologisch abbaubare Polymer P Gelatine ist, das Material der Mikrosphären des porenbildenden Mittels A Polymethylmethacrylat ist und das Lösungsmittel S Aceton ist.

8. Verfahren nach einem der Ansprüche 1 bis 7, **dadurch gekennzeichnet, dass** es zusätzlich im Schritt f) einen Schritt des Einbringens eines Kupplungsmittels, vorzugsweise einer Organoalkoxysilanzusammensetzung, mehr bevorzugt 3-Glycidoxypropyltrimethoxysilan (GPTMS) und noch mehr bevorzugt 3-Glycidoxypropyltriethoxysilan (GPTES) umfasst.

9. Verfahren nach einem der Ansprüche 1 bis 8, **dadurch gekennzeichnet, dass** es nach dem Schritt d) und vor dem Schritt e) einen Schritt des Erweiterns der Verknüpfungen (4) durch Infiltrieren des Materials des porenbildenden Mittels A in dem Stapel der Mikrosphären des porenbildenden Mittels A mit einem Lösungsmittel S und/oder durch Erwärmen dieses Stapels umfasst.

10. Verfahren zur Herstellung eines Hybridmaterials, umfassend ein biologisch abbaubares Polymer P und ein bioaktives Glas M auf Basis von SiO₂ und CaO, optional P₂O₅ enthaltend und/ oder optional mit Strontium dotiert, **dadurch gekennzeichnet, dass** es die folgenden Schritte umfasst:
A) Zubereitung eines Untergrunds von Alkoxid-Vorläufern eines bioaktiven Glases M auf Basis von SiO₂ und CaO, optional P₂O₅ enthaltend und/ oder optional mit Strontium dotiert,
B) Zufügen einer Lösung, das biologisch abbaubare Polymer P enthaltend, das in einem Lösungsmittel S gelöst ist, in den Untergrund des Schritts A),
C) Sol-Gel-Polymerisation der Alkoxid-Vorläufer und Gelieren des in Schritt B) erhaltenen Gemisches bei einer Temperatur im Bereich zwischen 0 °C und 60 °C, unter Luft.

11. Implantat-Material, das durch das Verfahren nach den Ansprüchen 1 bis 9 erhalten wird, zum Auffüllen von Knochendefekten, zur Knochenregeneration und zur Knochengewebezüchtung,
**dadurch gekennzeichnet, dass es ein Hybridmaterial umfasst, umfassend:**
• ein bioaktives Glas M auf Basis von SiO₂ und CaO, optional P₂O₅ enthaltend und/oder optional mit Strontium dotiert, und
• ein biologisch abbaubares Polymer P, das in mindestens einem Lösungsmittel S1 löslich ist, ausgewählt aus:
- biologisch resorbierbaren Polysacchariden, vorzugsweise ausgewählt aus Dextran, Hyaluronsäure, Agar, Chitosan, Alginsäure, Natrium- oder Kaliumalginat, Galaktomannan, Carrageen, Pektin,
- biologisch resorbierbaren Polyestern, vorzugsweise Polyvinylalkohol oder Poly(Milchsäure),
- biologisch abbaubaren synthetischen Polymeren, vorzugsweise einem Polyethylenglycol oder Poly(Caprolacton),
- Proteinen, vorzugsweise Gelatine oder Kollagen,
**und dadurch, dass** es aus einer Matrix (1) besteht, die das Hybridmaterial umfasst, wobei diese Matrix mindestens 70 % der Anzahl an Poren (2) aufweist, die die Form von Sphären oder Polyedern aufweisen, die in eine Sphäre eingeschrieben werden können, wobei der Durchmesser der Sphären im Bereich zwischen 100 und 900 µm, vorzugsweise zwischen 200 und 800 µm liegt, wobei eine Abweichung zwischen dem Durchmesser der kleinsten oder der größten Sphäre höchstens 70 %, vorzugsweise höchstens 50 %, mehr bevorzugt höchstens 30 % bezogen auf den durchschnittlichen arithmetischen Durchmesser der Gesamtheit der Sphären des Implantats beträgt und die Verknüpfungen (4) zwischen den Poren ihre kleinste Abmessung im Bereich zwischen 25 µm und 250 µm aufweisen, einschließlich der Extreme, wobei mindestens 70 % der Anzahl von Poren (2) mindestens eine Verknüpfung mit mindestens einer anderen Pore aufweisen.

12. Implantat aus einem Hybridmaterial zum Auffüllen von Knochendefekten, zur Knochenregeneration und zur Knochengewebezüchtung, **dadurch gekennzeichnet, dass** es ein Material nach Anspruch 11 umfasst.

## Claims

1. Method for manufacturing an implant made of a hybrid material for filling bone defects, for bone regeneration and for bone tissue engineering, **characterised in that** it comprises the following steps:
a) selecting a bioactive glass M based on SiO₂ and CaO, optionally containing P₂O₅ and/or optionally doped with strontium,
b) selecting a biodegradable polymer P which is soluble in at least one solvent S1 and insoluble in at least one solvent S different from the solvent S1,
c) selecting microspheres of a porogenic agent A that have diameters and sizes that correspond to the diameters and sizes sought for the pores in the material forming the implant to be manufactured, this porogenic agent A being:
- made of a polymer that is insoluble in the at least one solvent S1 and soluble in the at least one solvent S,
the at least one solvent S wherein the material of the biodegradable polymer P is insoluble and the at least one solvent S wherein the material of the porogenic agent A is soluble being identical,
d) introducing microspheres of the porogenic agent A into a mould that has the shape and the size sought for the implant, these microspheres forming a compact stack that corresponds to the shape and the size of the pores to be obtained in the implant material, and representing at least 60% by volume, preferably 70% by volume with respect to the total volume of the porogenic agent A-biodegradable polymer P-alkoxide precursors of the bioactive glass M mixture,
e) introducing the biodegradable polymer P into the alkoxide precursors of the bioactive glass M,
f) introducing the mixture obtained in step e) into the mould,
g) gelling the mixture contained in the mould after step f),
h) unmoulding the mixture obtained in step g),
i) removing microspheres of porogenic agent A by washing with the solvent S.

2. Method according to claim 1, **characterised in that** step e) and/or step f) are implemented before step d).

3. Method according to claim 1, **characterised in that** steps d) and e) and f) are implemented simultaneously.

4. Method according to any one of claims 1 to 3, **characterised in that** it comprises in addition a step j) of crosslinking the material obtained in step i).

5. Method according to any one of claims 1 to 4, **characterised in that** the biodegradable polymer P is chosen from:
- biodegradable polymers soluble in at least one solvent S1 and insoluble in at least one solvent S chosen from:
- bioresorbable polysaccharides, preferably chosen from dextran, hyaluronic acid, agar, chitosan, alginic acid, sodium or potassium alginate, galactomannan, carrageenan, pectin,
- bioresorbable polyesters, preferably polyvinyl alcohol or polylactic acid,
- biodegradable synthetic polymers, preferably a polyethylene glycol or polycaprolactone,
- proteins, preferably gelatin or collagen,
and **in that** the material of the porogenic agent A is chosen from the biodegradable polymers insoluble in the at least one solvent S1 and soluble in the at least one solvent S, preferably chosen from C₁ to C₄ alkyl polymethacrylates, preferably methyl polymethacrylate or butyl polymethacrylate, polyurethane, polyglycolic acid, the various forms of polylactic acids, the copolymers of lactic-coglycolic acids, polycaprolactone, polypropylene fumarate, paraffin and naphthalene, or acrylonitrile butadiene styrene (ABS).
the material of the porogenic agent A being different from the biodegradable polymer P.

6. Method according to any one of claims 1 to 5, **characterised in that** the biodegradable polymer P/ bioactive glass M ratio by weight is between 20/80 and 80/20, inclusive.

7. Method according to any one of claims 1 to 6, **characterised in that** the bioactive glass M is a glass based on SiO₂ and on CaO, the biodegradable polymer P is gelatin, the material of the microspheres of the porogenic agent A is methyl polymethacrylate and the solvent S is acetone.

8. Method according to any one of claims 1 to 7, **characterised in that** it comprises in addition, in step f), a step of introducing a coupling agent, preferably an organoalkoxysilane compound, more preferably 3-glycidoxypropyltrimethoxysilane (GPTMS), and even more preferably 3-glycidoxypropyltriethoxysilane (GPTES).

9. Method according to any one of claims 1 to 8, **characterised in that** it comprises after step d), and before step e), a step of enlarging the interconnections (4), by infiltration of a solvent S of the material of the porogenic agent A, in the stack of the microspheres of the porogenic agent A and/or by heating this stack.

10. Method for manufacturing a hybrid material comprising a biodegradable polymer P and a bioactive glass M based on SiO₂ and CaO and optionally containing P₂O₅ and/or optionally doped with strontium, **characterised in that** it comprises the following steps:
A) preparing a sol of alkoxide precursors of a bioactive glass M based on SiO₂ and on CaO, optionally containing P₂O₅ and/or optionally doped with strontium,
B) adding a solution containing the biodegradable polymer P, dissolved in a solvent S, in the sol of step A),
C) sol-gel polymerisation of the alkoxide precursors and gelling of the mixture obtained in step B) at a temperature between 0°C and 60°C, under air.

11. Implant material, obtained by the method described in claims 1 to 9, for filling bone defects, for bone regeneration and for bone tissue engineering,
**characterised in that** it comprises a hybrid material comprising:
• a bioactive glass M based on SiO₂ and CaO, optionally containing P₂O₅ and/or optionally doped with strontium, and
• a biodegradable polymer P soluble in at least one solvent S1 chosen from:
- bioresorbable polysaccharides, preferably chosen from dextran, hyaluronic acid, agar, chitosan, alginic acid, sodium or potassium alginate, galactomannan, carrageenan, pectin,
- bioresorbable polyesters, preferably polyvinyl alcohol or polylactic acid,
- biodegradable synthetic polymers, preferably a polyethylene glycol, polycaprolactone, and
- proteins, preferably gelatin or collagen,
and **in that** it consists of a matrix (1) comprising the hybrid material, this matrix having at least 70% by number of pores (2) having the shape of spheres or of polyhedrons inscribed within a sphere, with the diameter (3) of the spheres being comprised between 100 and 900 µm, preferably between 200 and 800 µm, inclusive, with a deviation between the diameter of the smallest sphere or the largest being at most 70%, preferably at most 50%, more preferably at most 30%, with respect to the arithmetic mean diameter of all the spheres of the implant and the interconnections (4) between the pores having their smallest dimension between 25 µm and 250 µm, inclusive, at least 70% by number of pores (2) having at least one interconnection with at least one other pore.

12. Implant made of a hybrid material for filling bone defects, for bone regeneration and for bone tissue engineering, **characterised in that** it comprises a material according to claim 11.
